# EUROPÄISCHE PATENTSCHRIFT

(11) **EP 1 525 215 B1**
(45) Veröffentlichungstag und Bekanntmachung des Hinweises auf die Patenterteilung: **06.09.2006**
(21) Anmeldenummer: 03784154.1
(22) Anmeldetag: 01.08.2003
(51) Int. Cl.: C07J 41/00, A61K 31/565, A61P 5/24

(54) **PROGESTERONREZEPTORMODULATOREN MIT ERHÖHTER ANTIGONADOTROPER AKTIVITÄT FÜR DIE WEIBLICHE FERTILITÄTSKONTROLLE UND HORMONERSATZTHERAPIE**
PROGESTERONE RECEPTOR MODULATORS HAVING AN INCREASED ANTIGONADOTROPIC ACTIVITY FOR USE IN FEMALE FERTILITY TESTING AND HORMONE REPLACEMENT THERAPY
MODULATEURS DE RECEPTEURS DE PROGESTERONE PRESENTANT UNE ACTIVITE ANTIGONADOTROPE AUGMENTEE, DESTINES AU CONTROLE DE FERTILITE ET AU TRAITEMENT HORMONAL SUBSTITUTIF CHEZ LA FEMME

(30) Priorität: 02.08.2002 DE 10236405; 02.08.2002 US 400065 P
(43) Veröffentlichungstag der Anmeldung: 27.04.2005
(73) Patentinhaber: Schering Aktiengesellschaft, 13353 Berlin (DE)
(72) Erfinder: HILLISCH, Alexander, 07743 Jena (DE); ELGER, Walter, 14195 Berlin (DE); SCHUBERT, Gerd, 07743 Jena (DE); SCHNEIDER, Birgitt, 07745 Jena (DE); REDDERSEN, Gudrun, 07749 Jena (DE)
(74) Vertreter: Rasch, Dorit
(86) Internationale Anmeldenummer: PCT/EP2003/008572
(87) Internationale Veröffentlichungsnummer: WO 2004/014935

(56) Entgegenhaltungen:
- EP-A- 0 648 778
- EP-A- 0 648 779
- EP-A- 0 909 764
- WO-A-01/15679
- WO-A-01/26603
- WO-A-01/34126
- WO-A-01/44267
- WO-A-99/45023
- DE-A- 3 504 421

## Beschreibung

Die Erfindung betrifft selektive Progesteronrezeptormodulatoren (SPRM), die zur Behandlung gynäkologischer Störungen, speziell zur hormonellen weiblichen Kontrazeption bzw. in der Hormonersatztherapie, eingesetzt werden können.

Progesteron wird während des Zyklus und in der Gravidität in großen Mengen vom Ovar bzw. der Plazenta sezemiert. Im Zusammenwirken mit Estrogenen bewirkt Progesteron zyklische Veränderungen der Utervsschleirnhaut im Menstruationszyklus. In der Gravidität kontrolliert Progesteron die Ruhigstellung des Myometriums und erhält die Funktion des dezidualen Gewebes.
Unter dem Einfluss erhöhter Progesteronspiegel nach der Ovulation wird die Uterusschleimhaut in einen Zustand überführt, der die Einnistung eines Embryos (Blastozyste) zulässt
Progesteron ist in subtiler Weise an der Steuerung von Ovulationsvorgängen beteiligt. Es ist bekannt, dass Progesteron in Verbindung mit Estrogenen antiovulatorische Eigenschaften besitzt. Diese resultieren aus einer Hemmung der hypophysären Gonadotropinsekretion, die Voraussetzung für die Reifung eines Follikels und dessen Ovulation ist. Andererseits ist erkennbar, dass die vergleichsweise geringe Progesteronsekretion des reifenden Follikels eine aktive Rolle für die Vorbereitung und Auslösung der Ovulation spielt. Hierbei spielen hypophysäre Mechanismen (zeitlich begrenzter sogenannter positiver feed back des Progesterons auf die Gonadotropinsekretion) eine erhebliche Rolle (D. Loutradie, Human Reproduction 6, 1991, 1238-1240).

Es ist weiterhin bekannt, dass Progesteron einen entscheidenden Einfluss auf das Endometrium ausübt. Durch die Unterdrückung der estrogen-vermittelten Mitose im Uterusgewebe wird die endometriale Proliferation gehemmt (K. Chwalisz, R.M. Brenner, U. Fuhrmann, H. Hess-Stumpp, W. Elger, Steroids 65, 2000,741-751).

Hormonelle Kontrazeption kann als Kombination aus antiovulatorischer Strategie verbunden mit dem Ersatz von fehlenden endogenen Hormonen betrachtet werden. Die konventionelle hormonelle Kontrazeption besteht darin, durch eine Kombination aus Gestagenen und Estrogenen den natürlichen Zyklus der Frau nachzuahmen, indem in 28-tägigem Rhythmus eine Entzugsblutung induziert wird. Abgesehen von ihrer Effektivität zeichnet sich diese Methode durch weitere Vorteile aus, die in einer Reduzierung des kanzerogenen Risikos, speziell des Ovarial- bzw. des Endometriumkrebses, bzw. ihrem positiven Einfluss auf das kardiovaskuläre System zu sehen sind.
Ein Nachteil der konventionellen hormonellen Kontrazeption ist mit der Verabreichung relativ hoher Dosen von Estrogenen (i.a. Ethinylestradiol, EE) verbunden, welche essentiell für ein reguläres Blutungsverhalten sind. Estrogene, insbesondere Ethinylestradiol, beeinflussen deutlich bestimmte Leberfunktionen wie z.B. die Synthese der Transportproteine CBG, SHBG, TBG, Angiotensinogen, weiterhin verschieden Funktionen, die in der Physiologie der Blutgerinnung eine Rolle spielen sowie die Lipoproteine (HDL, LDL).
Die starke Estrogenwirkung des EE in der Leber, aber auch der natürlichen Estrogene während der Gravidität, finden ihren Ausdruck u.a. in einem erhöhten thromboembolischen Risiko. Auch ohne die pathophysiologischen Grundlagen thrombo-embolischer Komplikationen im Einzelnen zu kennen, kann man davon ausgehen, dass hepatischen Estrogenwirkungen hierbei eine entscheidende Rolle zukommt.

Selektive Progesteronrezeptormodulatoren, auch Mesoprogestine genannt, sind eine neue Klasse von Progesteronrezeptor(PR)-Liganden, welche am Progesteronrezeptor *in vivo* sowohl agonistische als auch antagonistische Wirkung zeigen.
Dies führt zu einer Neutralisation der sowohl bei reinen PR-Agonisten als auch bei reinen PR-Antagonisten vorhandenen antiovulatorischen. Wirksamkeit [Slayden, OD, Chwalisz K and Brenner RM Reversible suppression of menstruation with progesterone antagonists in rhesus macaques. *Hum Reprod* 16:1562-1574,.2001, Elger W, Bartley J, Schneider B, Kaufmann G, Schubert G, Chwalisz K. Endocrine pharmacological characterization of progesterone antagonists and progesterone receptor modulators with respect to PR-agonistic and antagonistic activity. Steroids 65, 713-723 (2000)]. Fehlende oder unsichere inhibitorische Effekte auf die Ovulation werfen Fragen zur antifertilen Wirksamkeit von entsprechenden Mesoprogestinen auf.
Ein weiteres Merkmal der Mesoprogestine ist das gegenüber der nahezu ausschließlich als Antigestagen wirkenden Verbindung RU 38 468 reduzierte bzw. fehlende Potential der Verbindungen, einen Abort auszulösen.

Chemische Verbindungen der nachstehend gezeigten Struktur, worin R ein Wasserstoffatom oder eine Alkylgruppe und R¹ ein Wasserstoffatom, eine Alkyl- oder Arylgruppe bzw. eine gegebenenfalls substituierte Acylfunktion sein kann, sind als Mesoprogestine bekannt:

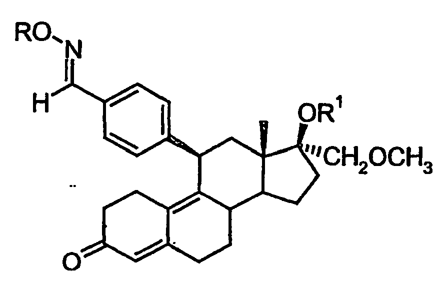

***WO 01*/*44267*** beschreibt neue 11β-Phenylestradien-derivate mit Fluoralkylgruppen in der aromatischen Seitenkette und deren Herstellung. Die Verbindungen bzw. diese Verbindungen enthaltende pharmazeutische Präparate sind antihormonell wirksam und daher für die Behandlung von Erkrankungen, die durch Cortisol bzw. durch Corticoide ungünstig beeinflusst werden, zur Reduktion des sezemierten Cortisols, zur Stimulation der Laktation, zur Behandlung der Dysmenorrhoe und von Myomen, zur Behandlung von Morbus Cushing und zur Zervixreifung, zur Verbesserung der kognitiven Leistungen, zur Behandlung der Endometriose oder für die Hormon Replacement Therapie (HRT) geeignet.

***WO99*/*45023*** betrifft S-substituierte 11β-Benzaldoxim-estra-4,9-dien-kohlensäure-thiolester. Die Verbindungen besitzen antigestagene Eigenschaften bei gleichzeitig im Vergleich zu RU 468 deutlich reduzierter antiglucocorticoider Wirkung.

In ***EP 909764*** sind 11β-Benzaldoxim-9α,10α-epoxy-estr-4-en-derivate mit hoher Bindungsaffinität zum Progesteronrezeptor bei geringer Glucocorticoidrezeptoraffinität beschrieben.

***DE 4332283*** und ***US 5693628*** betreffen 11-Benzaldoxim-estra-4,9-dien-derivate der allgemeinen Formel 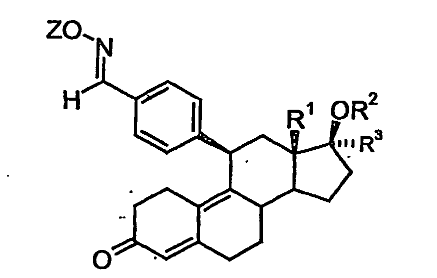 in der
R¹ ein Wasserstoffatom oder eine Alkylgruppe ist,
R² ein Wasserstoffatom, ein Alkyl-, Aryl-, Aralkyl-, Alkylarylrest, eine Gruppe CONHR⁴, CO₂R⁴ ist, worin R⁴ ein Wasserstoffatom, eine Alkyl-, Aryl-, Aralkyl- oder Alkylarylgruppe mit bis zu 10 Kohlenstoffatomen sein kann,
R³ ein Wasserstoffatom oder eine Alkylgruppe ist, wobei die Alkylgruppe aus 1-6 Kohlenstoffatomen bestehen kann bzw. R³ einen Rest (CH₂)ₙCH₂X mit n = 0, 1 oder 2 und X Fluor, Chlor, Brom oder lod, bzw. Cyano, Azido oder Rhodano oder einen Rest OR⁵ oder SR⁵ bedeuten kann.
Z kann sowohl Wasserstoff als auch verschiedene andere Substituenten bedeuten.

Die Verbindungen 4-[17β-Hydroxy-3-oxoestra-4,9-dien-11β-yl]benzaldehyd-1-(E)-oxim und 4-[17β-Hydroxy-17α-methyl-3-oxoestra-4,9-dien-11β-yl]benz-aldehyd-1-(E)-oxim sind nicht explizit genannt.

Die offenbarten Verbindungen zeichnen sich durch eine stark antigestagene Wirkung bei reduzierter glucocorticoider Aktivität aus.

In der nicht vorveröffentlichten Anmeldung ***DE 102 1034*** werden 17α-Fluoralkyl-11 β-benzaldoxim-Steroide der allgemeinen Formel 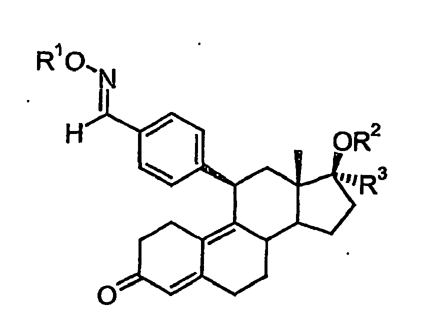 beschrieben, worin
R¹ für Wasserstoff, C₁- bis C₆-Alkyl, COR⁴, COOR⁴, COSR⁴ oder CONHR⁵ steht, worin R⁴ C₁- bis C₆-Alkyl oder unsubstituiertes oder substituiertes Aryl ist und worin R⁵ Wasserstoff, C₁- bis C₆-Alkyl oder unsubstituiertes oder substituiertes Aryl ist,
R² für Wasserstoff, C₁- bis C₆-Alkyl oder C₁- bis C₆-Acyl steht und R³ für eine CₙF₂ₙ₊₁-Gruppe, bei der n = 1, 2 oder 3 ist, oder eine CH₂(CH₂)ₘCₙF₂ₙ₊₁ steht, bei der m = 0 oder 1 und n =1, 2 oder 3 sind,
als antigestagen wirksame Verbindungen mit deutlich reduzierter antiglucocorticoider Wirkung im Vergleich zu RU 38486.
Aufgabe der vorliegenden Erfindung ist es, Verbindungen für die weibliche Kontrazeption zur Verfügung zu stellen, die bekannte Vorteile der konventionellen Kontrazeption und zusätzliche Vorteile in sich vereinen. Die mit den erfindungsgemäßen Verbindungen realisierbare kontrazeptive Methode soll jedoch vorzugsweise ohne den Zusatz von exogenen Estrogenen auskommen. In konventionellen Kontrazeptiva sind die eingesetzten Dosen von EE zur Erreichung einer sicheren Unterdrückung der ovariellen Funktion und zur Erhaltung eines menstruellen Blutungsmusters essentiell. Die erfindungsgemäßen Verbindungen sollen sich durch Weglassen oder durch Reduktion der Dosis der Estrogenkomponente durch ein geringeres thromboembolisches Nebenwirkungspotential auszeichnen. Durch die Verbindungen soll eine Amenorrhoe induziert werden können. Gleichzeitig soll eine Stimulation der Brustdrüse vermieden werden. Besonders vorteilhafte Verbindungen sollen in der Lage sein, sowohl estrogene als auch gestagene Effekte im Endometrium zu unterdrücken. Die erfindungsgemäße Verbindungen sollen die Ovulation hemmen und die Knochensubstanz in Abwesenheit von Estrogenen erhalten, sei es in der postmenopausalen Frau oder nach Unterdrückung der ovariellen Estrogensekretion prämenopausal.

Die Aufgabe wird gemäß vorliegender Erfindung gelöst durch die Verwendung von Verbindungen der allgemeinen Formel I 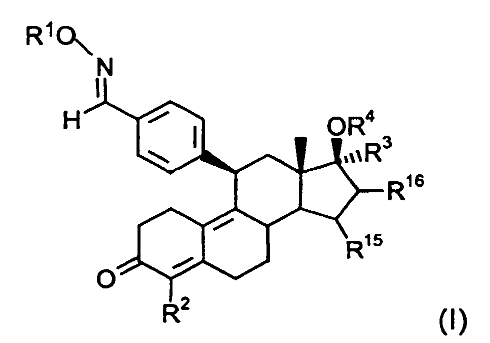 worin die Reste R¹, R², R³, R⁴ und R⁵ sowie R¹⁵ und R¹⁶ folgende Bedeutung besitzen:
- R¹: ein Wasserstoffatom, ein Alkanoylrest mit 1 bis 10 Kohlenstoffatomen oder ein gegebenenfalls substituierter Benzoylrest mit 6-10 Kohlenstoffatomen oder ein Rest CONHR⁵, wobei
R⁵ ein Wasserstoffatom, ein Alkyl- oder Acylrest mit jeweils 1-10 Kohlenstoffatomen oder ein Alkylaryl- oder Aralkylrest mit jeweils 6-10 Kohlenstoffatomen ist,
- R²: ein Wasserstoffatom, ein Halogenatom oder eine CF₃-Gruppe,
- R³: ein Wasserstoffatom oder eine Gruppe CH₂X, in der
X für ein Wasserstoffatom, eine Hydroxygruppe, ein Halogenatom, einen Alkylrest mit 1 oder 2 Kohlenstoffatomen, für einen Rest (CH₂)ₙCH₂Y mit n = 0 oder 1 und Y für ein Halogenatom steht, wobei, wenn
R² ein Halogenatom ist, R³ zusätzlich eine Gruppe CₙFₘHₒ bedeuten kann, wobei n = 1, 2, 3, 4 oder 5, m > 1 und
m + o = 2n + 1 ist,
- R⁴: ein Wasserstoffatom, Alkyl- oder Alkanoylrest aus jeweils 1-10 Kohlenstoffatomen oder ein Benzoylrest mit 6-10
Kohlenstoffatomen oder ein Rest -CONHR⁵, wobei R⁵ die oben angegebene Bedeutung besitzt,
R¹⁵ und R¹⁶ Wasserstoffatome oder gemeinsam eine Doppelbindung darstellen,
sowie iher pharmazeutisch annehmbaren Salze,
zur Herstellung von Arzneimitteln zur Behandlung von dysfunktionellen Blutungen, zur Behandlung von Dysmenorrhoe, zur Induktion einer Amenorrhoe, zur Behandlung von hormonellen Störungen bei postmenopausalen Frauen zur weiblichen Fertilitätskontrolle und die Bereitstellung von neuen 11β-Benzaldoxim-estra-4,9-dien-Derivaten der allgemeinen Formel I 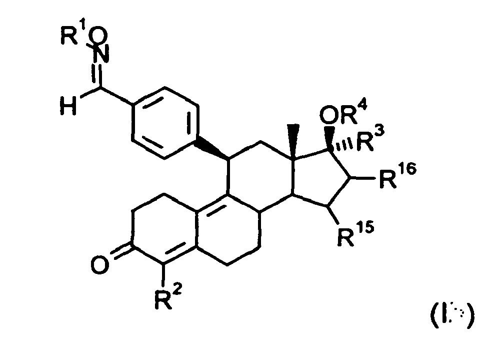 worin die Reste R¹, R², R³, R⁴ und R⁵ sowie R¹⁵ und R¹⁶ folgende Bedeutung besitzen:
R¹ ein Wasserstoffatom, ein Alkanoylrest mit 1 bis 10 Kohlenstoffatomen oder ein gegebenenfalls substituierter Benzoylrest mit 6-10 Kohlenstoffatomen oder ein Rest CONHR⁵, wobei
R⁵ ein Wasserstoffatom, ein Alkyl- oder Acylrest mit jeweils 1-10 Kohlenstoffatomen oder ein Alkylaryl- oder Aralkylrest mit jeweils 6-10 Kohlenstoffatomen ist,
R² ein Halogenatom oder eine CF₃-Gruppe,
R³ ein Wasserstoffatom oder eine Gruppe CH₂X, in der X für ein Wasserstoffatom, eine Hydroxygruppe, ein Halogenatom, einen Alkylrest mit 1 oder 2 Kohlenstoffatomen, für einen Rest (CH₂)ₙCH₂Y mit n = 0 oder 1 und Y für ein Halogenatom steht, wobei, wenn
R² ein Halogenatom ist, R³ zusätzlich eine Gruppe CₙFₘHₒ bedeuten kann, wobei n = 1, 2, 3, 4 oder 5, m > 1 und
m + o = 2n + 1 ist,
R⁴ ein Wasserstoffatom, ein Alkyl- oder Alkanoylrest aus jeweils 1-10 Kohlenstoffatomen oder ein Benzoylrest mit 6-10 Kohlenstoffatomen oder ein Rest -CONHR⁵, wobei R⁵ die oben angegebene Bedeutung besitzt,
R¹⁵ und R¹⁶ Wasserstoffatome oder gemeinsam eine Doppelbindung darstellen,
sowie ihrer pharmazeutisch annehmbaren Salze.

Weiterhin umfasst die vorliegende Erfindung die neuen Substanzen als pharmazeutische Wirkstoffe, deren Herstellung, ihre therapeutische Anwendung und die pharmazeutischen Darreichungsformen, die die neuen Substanzen enthalten.

Die erfindungsgemäßen Verbindungen der allgemeinen Formel (I) oder deren pharmazeutisch annehmbare Salze können für die Herstellung eines Arzneimittels zur Verwendung in der weiblichen Fertilitätskontrolle sowie zur Behandlung der Endometriose sowie des Uterus myomatoses eingesetzt werden.

Für die HRT können die Verbindungen allein oder in Verbindung mit einem natürlichen Estrogen (z. B. Estradiol, dessen Ester, Estron, Estronsulfat, Estriol und Prodrugs dieser Estrogene) eingesetzt werden.

R² ist vorzugsweise ein Chlor- oder Bromatom.
Für die Substituenten X und Y kann ein Halogenatom Fluor, Chlor oder Brom bedeuten.

Wenn nicht näher definiert, handelt es sich im Sinne der vorliegenden Erfindung bei einem Arylrest, der gegebenenfalls substituiert sein kann, um einen Phenyl-, 1- oder 2-Naphthylrest, wobei der Phenylrest bevorzugt ist. Wenn nicht ausdrücklich erwähnt, schließt Aryl immer auch einen Heteroarylrest mit ein. Beispiele für einen Heteroarylrest sind der 2-, 3- oder 4-Pyridinyl-, der 2- oder 3-Furyl, der 2- oder 3-Thienyl, der 2- oder 3-Pyrrolyl-, der 2-, 4- oder 5-Imidazolyl-, der Pyrazinyl-, der 2-, 4- oder 5-Pyrimidinyl- oder 3- oder 4-Pyridazinylrest.

Unter Alkylresten sind gerad- oder verzweigtkettige, gesättigte oder ungesättigte Alkylreste zu verstehen. Als Vertreter für gerad- oder verzweigtkettige Alkylgruppen mit 1-5 bzw. 1-10 Kohlenstoffatomen sind beispielsweise Methyl, Ethyl, Propyl, Isopropyl, Butyl, Isobutyl, tert.-Butyl, Pentyl, Isopentyl, Neopentyl, Heptyl, Hexyl, Decyl zu nennen; Methyl, Ethyl, Propyl und Isopropyl sind bevorzugt.

Bevorzugt sind Verbindungen, in denen R¹ ein Wasserstoffatom bedeutet, R² für ein Wasserstoff-, ein Chlor- oder ein Bromatom steht und R³ ein Wasserstoffatom, eine Methylgruppe, eine CH₂X-Gruppe sein kann, wobei X für ein Fluor-, Chlor oder Bromatom oder eine Hydroxygruppe steht. Die Substituenten R⁴, R¹⁵ und R¹⁶ haben die in der allgemeinen Formel I angegebene Bedeutung.

Bevorzugt sind Verbindungen, in denen R⁴ ein Wasserstoffatom oder ein Alkylrest mit 1 bis 4 Kohlenstoffatomen ist.

Besonders bevorzugt sind nachstehend genannte Verbindungen bzw. deren erfindungsgemäße Verwendungen:
4-[4'-Brom-17β-hydroxy-3-oxoestra-4,9-dien-11β-yl]benzaldehyd-1-(E)-oxim,
4-[4'-Brom-17β-hydroxy-17α-methyl-3-oxoestra-4,9-dien-11β-yl]benzaldehyd-1-(E)-oxim,
4-[4'-Brom-17β-hydroxy-17α-trifluormethyl-3-oxoestra-4,9-dien-11β-yl]benzaldehyd-1-(E)-oxim,
4-[17α-Brommethyl-17β-hydroxy-3-oxoestra-4,9-dien-11β-yl]benzaldehyd-1-(E)-oxim,
4-[17β-Hydroxy-3-oxoestra-4,9-dien-11β-yl]benzaldehyd-1-(E)-oxim,
4-[17β-Acetoxy-3-oxoestra-4,9-dien-11β-yl]benzaldehyd-1-(E)-oxim,
4-[17β-Acetoxy-4'-brom-3-oxoestra-4,9-dien-11β-yl]benzaldehyd-1-(E)-oxim,
4-[17β-Acetoxy-4'-brom-3-oxoestra-4,9-dien-11β-yl]benzaldehyd-1-(E)-O-acetyloxim,
4-[17β-Benzoyloxy-3-oxoestra-4,9-dien-11β-yl]benzaldehyd-1-(E)-oxim,
4-[17β-(N-Ethylamino)carbonyloxy-3-oxoestra-4,9-dien-11β-yl]benzaldehyd-1-(E)-oxim,
4-[17β-Hydroxy-3-oxoestra-4,9-dien-11β-yl]benzaldehyd-1-(E)-[N-(ethylamino)-carbonyl]oxim,
4-[17β-Methoxy-3-oxoestra-4,9-dien-11β-yl]benzaldehyd-1-(E)-[N-(ethylamino)-carbonyl]oxim,
4-[17β-Methoxy-3-oxoestra-4,9-dien-11β-yl]benzaldehyd-1-(E)-oxim,
4-[4'-Brom-17β-methoxy-3-oxoestra-4,9-dien-11β-yl]benzaldehyd-1-(E)-oxim,
4-[17β-Hydroxy-3-oxoestra-4,9,15-trien-11β-yl]benzaldehyd-1-(E)-oxim,
4-[17β-Methoxy-3-oxoestra-4,9,15-trien-11β-yl]benzaldehyd-1-(E)-oxim,
4-[17β-Hydroxy-17α-methyl-3-oxoestra-4,9-dien-11β-yl]benzaldehyd-1-(E)-oxim,
4-[4'-Chlor-17β-hydroxy-17α-trifluormethyl-3-oxoestra-4,9-dien-11β-yl]benzaldehyd-1-(E)-oxim,
4-[4'-Chlor-17β-hydroxy-3-oxoestra-4,9-dien-11β-yl]benzaldehyd-1-(E)-oxim
4-[17α-Fluormethyl-17β-hydroxy-3-oxoestra-4,9-dien-11β-yl]benzaldehyd-1-(E)-oxim,
4-[4'-Brom-17α-fluormethyl-17β-hydroxy-3-oxoestra-4,9-dien-11β-yl]benzaldehyd-1-(E)-oxim,
4-[4'-Brom-17α-chlormethyl-17β-hydroxy-3-oxoestra-4,9-dien-11β-yl]benzaldehyd-1-(E)-oxim,
4-[4'-Brom-17α-brommethyl-17β-hydroxy-3-oxoestra-4,9-dien-11β-yl]benzaldehyd-1-(E)-oxim,
4-[4'-Chlor-17β-methoxy-3-oxoestra-4,9-dien-11β-yl]benzaldehyd-1-(E)-oxim,
4-[4'-Chlor-17α-chlormethyl-17β-hydroxy-3-oxoestra-4,9-dien-11β-yl]benzaldehyd-1-(E)-oxim,
4-[17β-Ethoxy-3-oxoestra-4,9-dien-11β-yl]benzaldehyd-1-(E)-oxim,
4-[17β-Isopropyloxy-3-oxoestra-4,9-dien-11β-yl]benzaldehyd-1-(E)-oxim,
4-[17β-Benzyloxy-3-oxoestra-4,9-dien-11β-yl]benzaldehyd-1-(E)-oxim,
4-[17β-Methoxy-4'-trifluormethyl-3-oxoestra-4,9-dien-11β-yl]benzaldehyd-1-(E)-oxim,
4-[4'-Chlor-17β-hydroxy-17α-methyl-3-oxoestra-4,9-dien-11β-yl]benzaldehyd-1(E)-oxim,
4-[17β-Hydroxy-17α-methyl-3-oxoestra-4,9-dien-11β-yl]benzaldehyd-1-(E)-[O-(ethylamino)carbonyl]oxim,
4-[17β-Hydroxy-17α-hydroxymethyl-3-oxoestra-4,9-dien-11β-yl]benzaldehyd-1-(E)-[O-(ethylamino)-carbonyl]oxim.

Für die Bildung von pharmazeutisch verträglichen Salzen der erfindungsgemäßen Verbindungen der allgemeinen Formel I kommen als anorganische Säuren unter anderem Chlorwasserstoffsäure, Bromwasserstoffsäure, Schwefelsäure und Phosphorsäure, sowie als organische Säuren unter anderem Essigsäure, Propionsäure, Maleinsäure, Fumarsäure, Bemsteinsäure, Benzoesäure, Ascorbinsäure, Oxalsäure, Salicyläure, Weinsäure, Zitronensäure, Milchsäure, Äpfelsäure, Mandelsäure, Zimtsäure und Methansulfonsäure in Betracht.

Die Verbindungen zeichnen sich durch eine milde androgene und gleichzeitig verbesserte antigonadotrope Wirksamkeit aus. Für die strukturell nächstliegenden Verbindungen, wie sie aus dem eingangs erwähnten Stand der Technik bekannt sind, wurde bisher keine androgene Wirksamkeit beschrieben. Die erfindungsgemäßen Substanzen gemäß allgemeiner Formel I stellen zudem Verbindungen dar, die eine gegenüber bekannten Verbindungen verbesserte agonistische Aktivität am Progesteronrezeptor aufweisen.

### Biologische Charakterisierung der erfindungsgemäßen Verbindungen

Die erfindungsgemäßen Substanzen der allgemeinen Formel I wurden in folgenden Modellen getestet:

### Rezeptorbindungstests

### Messung der Rezeptor-Bindüngsaffinität:

Die Rezeptorbindungsaffinität wurde bestimmt durch competitive Bindung eines spezifisch bindenden ³H-markierten Hormons (Tracer) und der zu testenden Verbindung an Rezeptoren im Cytosol aus tierischen Target-Organen. Dabei wurden Rezeptorsättigung und Reaktionsgleichgewicht angestrebt.
Der Tracer und steigende Konzentrationen der zu testenden Verbindung (Competitor) wurden bei 0-4°C über 18 h co-inkubiert mit der rezeptorhaltigen Cytosolfraktion. Nach Abtrennung des ungebundenen Tracers mit Kohle-Dextran-Suspension wurde für jede Konzentration der Rezeptor-gebundene
Tracer-Anteil gemessen und aus der Konzentrationsreihe die IC₅₀ bestimmt. Als Quotient der IC₅₀-Werte von Referenzsubstanz und zu testender Verbindung (x 100%) wurde die relative molare Bindungsaffinität (RBA) errechnet (RBA der Referenzsubstanz = 100%).

Für die einzelnen Rezeptortypen wurden folgende Inkubationsbedingungen gewählt

### Progesteron-Rezeptor:

Uterus-Cytosol des Estradiol-geprimten Kaninchens, homogenisiert in TED-Puffer (20 mMTris/HCl, pH 7,4; 1 mM Ethylendiamintetraacetat, 2 mM Dithiothreitol) mit 250 mM Saccharose; aufbewahrt bei -30 °C. Tracer: ³H- ORG 2058, 5 nM; Referenzsubstanz: Progesteron.

### Glucocorticoid-Rezeptor:

Thymus-Cytosol der adrenalectomierten Ratte, Thymi aufbewahrt bei -30°C; Puffer: TED. Tracer: ³H-Dexamethason, 20 nM; Referenzsubstanz:
Dexamethason.
Androgen-Rezeptor:
Prostata-Cytosol der kastrierten Ratte; Prostatae aufbewahrt bei -30°C; Puffer:
TED mit 10% Glycerol sowie 2 µM Triamcinolonacetonid. Tracer ³H-Metribolon 4 nM; Referenzsubstanzen: Metribolon (RU 1881) bzw. 5α-Dihydrotesto-steron.

**Tabelle 1**

| Bsp. | R₂ | Oxim | 17β | 17α | PR¹⁾ | GR²⁾ | AR³⁾ |
|---|---|---|---|---|---|---|---|
| 1 | H | H | OH | H | 26 | 3 | a) 17 |
| | | | | | | | b) 24 |
| 2 | H | H | OH | CH₃ | 95 | 12 | a) 16 |
| | | | | | | | b) 23 |
| 3 | H | H | OCOCH₃ | H | 20 | 12 | n.b. |
| 4 | H | H | OCONHEt | H | 13 | 11 | a) 2,9 |
| | | | | | | | b) 4,5 |
| 5 | H | H | OCH₃ | H | 159 | 14 | a) 21 |
| | | | | | | | b) 32 |
| 6 | Br | H | OH | CH₃ | 106 | 0,76 | a) 14 |
| | | | | | | | b) 22 |
| 7 | Br | H | OCH₃ | H | 110 | 0,69 | a) 8,7 |
| | | | | | | | b) 13,2 |
| 8 | Br | H | OH | CF₃ | 87 | 1,8 | a) 7,8 |
| | | | | | | | b) 11,8 |
| 9 | H | CONHEt | OH | CH₃ | 161 | 25 | a) 15 |
| | | | | | | | b) 23 |
| 10 | H | H | OH | CH₂Br | 21 | 3 | a) 0,6 |
| | | | | | | | b) 0,9 |
| 11 | H | CONHEt | OH | H | 34 | 10 | a) 10 |
| | | | | | | | b) 15 |
| 12⁴⁾ | H | H | OH | H | 38 | 5 | a) 22 |
| | | | | | | | b) 34 |
| 13 | Br | H | OCOCH₃ | H | 45 | 0,35 | a) 6 |
| | | | | | | | b) 10 |
| 14 | Br | COCH₃ | OCOCH₃ | H | 23 | 0,3 | a) 6 |
| | | | | | | | b) 10 |
| 15 | H | H | OC₂H₅ | H | 143 | 17 | a) 13 |
| | | | | | | | b) 21 |
| 16 | H | H | OCOPh | H | 8 | 3 | a) 1,4 |
| | | | | | | | b) 2,2 |
| 17 | H | H | OPh | H | 31 | 8 | a) 2 |
| | | | | | | | b) 3 |
| 19 | Br | H | OH | H | 51 | 0,5 | a) 9 |
| | | | | | | | b) 16 |
| 20 | H | CONHEt | OH | CH₂OH | 12 | 5 | a) 4 |
| | | | | | | | b) 7 |

| | | | | | | | |
|---|---|---|---|---|---|---|---|
| ¹⁾ Progesteron = 100 %, ²⁾ Dexamethason = 100 %, ³⁾ a) gegen RU 1881 (17β-Hydroxy-17α-methyl-estra-4,9,11-trien-3-on) und b) gegen Dihydrotestosteron (DHT), n.b. nicht bestimmt , ⁴) zusätzliche 15,16-Doppelbindung. | | | | | | | |

### Auswertung antifertiler Effekte der Substanzen in der Perinidationsphase der Ratte

Die Ermittlung der progesteronantagonistischen Aktivitäten erfolgte an adulten weiblichen Ratten im Nidationshemmtest.
Die Hemmung des Progesteronrezeptors führt in der sehr frühen Gravidität der Ratte zu starken antifertilen Effekten. Partial PR-agonistische Eigenschaften von Substanzen schwächen die negativen Wirkungen auf die Gravidität in dieser Phase des Reproduktionsgeschehens (Nidation) nicht ab (Elger W, Bartley J, Schneider B, Kaufmann G, Schubert G, Chwalisz K. Endocrine pharmacological characterization of progesterone antagonists and progesterone receptor modulators with respect to PR-agonistic and antagonistic activity. Steroids 65, 713-723 (2000)). Anders als reine PR-Antagonisten haben die erfindungsgemäßen Substanzen keine oder stark reduzierte Hemmeffekte auf die Gravidität im Sinne reduzierter oder aufgehobener Fähigkeit, Wehen auszulösen.
Das Prinzip des Versuches beschreibt sich wie folgt: Progesteron erhält die Gravidität in allen Stadien. Entsprechend kann von kompetitiven Progesteronantagonisten eine frühabortive Wirkung erwartet werden.
Weibliche Ratten (Stamm Schoe: WIST Tierzucht GmbH Schönwalde) mit einem Gewicht von 180 - 200g, wurden täglich einer Zykluskontrolle unterzogen und im Prooestrus angepaart. Der Beginn der Gravidität wurde durch Nachweis von Spermien im Vaginalabstrich am Tag darauf (= Tag 1 der Gravidität = d1 p.c.) festgestellt.
Vom 5. - 7. Tag der Gravidität wurden täglich die Testsubstanzen subkutan injiziert. Am 9. Tag erfolgte die Abnahme von Vaginalabstriche, anschließend wurden die Tiere authopsiert und die Uteri präpariert.
Die Rückbildung von Implantaten und pathologische, hämorrhagische und sonstige abnorme Nidationsstellen wurden als Abort gewertet und in Tabelle 2 dargestellt.

**Tabelle 2**

| | | |
|---|---|---|
| **Substanz** | **Dosierung mg/Tier/Tag** | **Rate der Tiere mit kompletter Hemmung der Nitation** |
| Vehikelkontrolle | 0,2 ml Benzylbenzoat + Ricinusöl (1+4) | 0/5 |
| RU 38 486 | 0.3 | 0/5 |
| | 1.0 | 2/5 |
| | 3.0 | 5/5 |
| Beispiel 1 | 1.0 | 0/5 |
| | 3.0 | 0/5 |
| | 10.0 | 5/5 |
| Beispiel 2 | 0.03 | 0/4 |
| | 0.1 | 0/5 |
| | 0.3 | 2/5 |
| | 1.0 | 5/5 |
| Beispiel 5 | 1.0 | 0/4 |
| Beispiel 6 | 1.0 | 5/5 |

Die Ergebnisse dieser Untersuchungen zeigen, dass erfindungsgemäße Mesoprogestine PR-antagonistische Eingeschaften besitzen.

### Antiluteolysetest/Ovulationshemmtest am zyklischen Meerschweinchen

Die Prüfung der Substanzen auf progesteronagonistische bzw. progesteronantagonistische Aktivität erfolgte im Antiluteolysetest an adulten weiblichen Meerschweinchen nach subkutaner Applikation.
Das Prinzip des Versuches stellt sich wie folgt dar: Die Rückbildung der Gelbkörper im nichtfertilen Zyklus erfolgt beim Meerschweinchen durch Prostaglandine (PGF₂α), die der Uterus freisetzt. In der Gravidität persistieren die Gelbkörper, da der Embryo die Prostaglandinsekretion des Uterus hemmt [Elger W, Bartley J, Schneider B, Kaufmann G, Schubert G, Chwalisz K. Endocrine pharmacological characterization of progesterone antagonists and progesterone receptor modulators with respect to PR-agonistic and antagonistic activity. Steroids 65, 713-723 (2000)]. Im Zyklus stimuliert das Progesteron die Sekretion von PGF2α des Uterus. Reine PR-Antagonisten hemmen diese Funktion vollständig (Hemmung der Luteolyse). Die vorliegende Versuchsanordnung ermöglicht es, die Hemmung der uterinen Prostaglandinfreisetzung als antiluteolytische Wirkung durch Progesterongehaltsbestimmungen im Serum zwischen den Tagen d0; d6/d7/d8und d10-d18 zu erfassen. Gleichzeitig kann die Ovulationshemmung durch histologische Beurteilung der Gelbkörper im Ovar ermittelt werden (neue Gelbkörper = Ovulation).

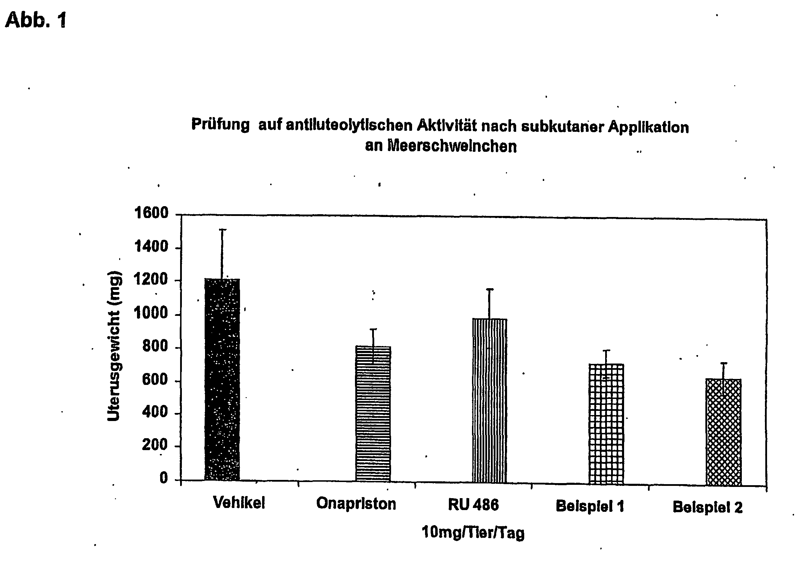

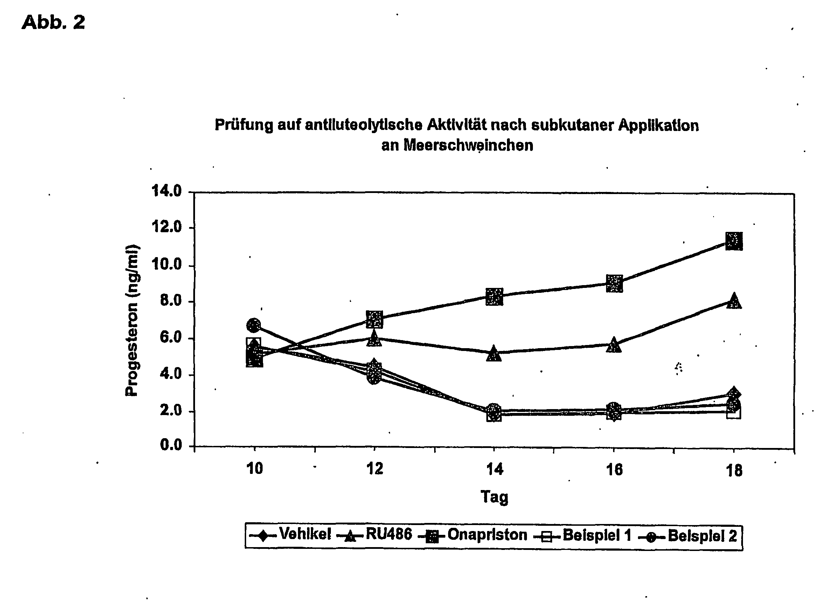

Weibliche Meerschweinchen (Stamm Schoe : DH, Tierzucht GmbH Schönwalde) mit einem Gewicht von 500g wurden von Tag 10 bis Tag 17 tägliche mit Prüfsubstanzen bzw. den Vergleichsubstanzen Onapriston und RU 486 in der Dosis von 10 mg/Tier/Tag subkutan behandelt. Die Kontrollgruppe erhielt das Substanzvehikel Benzylbenzoat / Ricinusöl appliziert. Das Applikationsvolumen betrug 0.2 ml/Tier/Tag, jede Gruppe umfasste 5 Versuchstiere.
An den Tagen 10, 12, 14, 16 und 18 wurde Blut für Progesterongehaltsbestimmung aus dem retroorbitalen Venenplexus entnommen. 24 Stunden nach der letzten Applikation wurden die Tiere authopsiert. Die Ergebnisse sind in Abbildung 1 und 2 dargestellt.
Substanzen mit Affinität zu PR und AR, die die Ovulation hemmen, aber nicht die Rückbildung der Gelbkörper sind erfindungsgemäße Mesoprogestine.

### Ermittlung der androgenen und antigonadotropen Eigenschaften der Substanzen im Hershberger Test an infantilen Ratten

Die Ermittlung der androgenen Eigenschaften erfolgte im Hershberger-Test an infantilen männlichen Ratten nach subkutaner Applikation über 7 Tage.

Das Prinzip des Versuches stellt sich wie folgt dar: Die Funktion und Größe der akzessorischen Geschlechtsdrüsen (Samenblasen und Prostata) und des *Musculus levator ani* sind abhängig von der Anwesenheit von Androgenen. Eine Kastration führt dementsprechend zur Atrophie dieser Organe. Substituiert man nach der Kastration mit einem Androgen (Testosteronpropionat), kann man durch die Gewichtszunahme der akzessorischen Drüsen auf eine androgene Aktivität von Substanzen schließen.

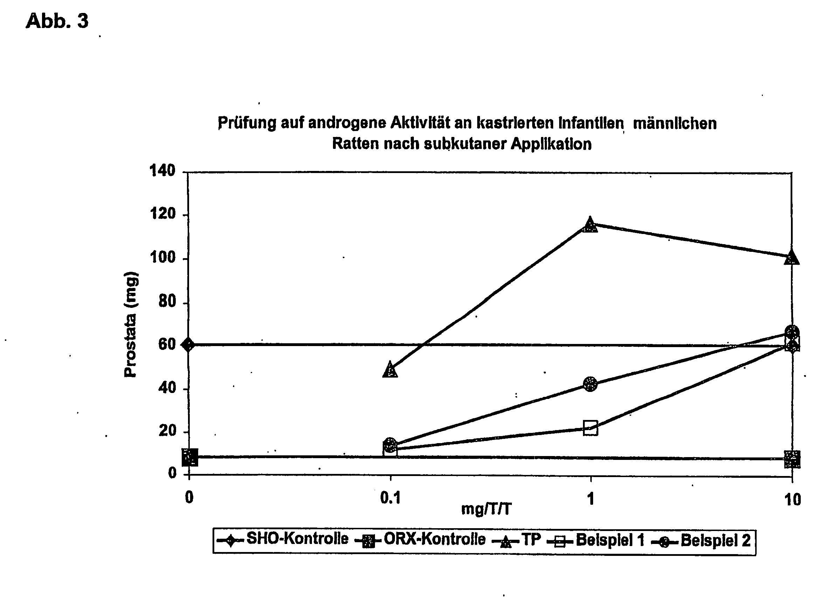

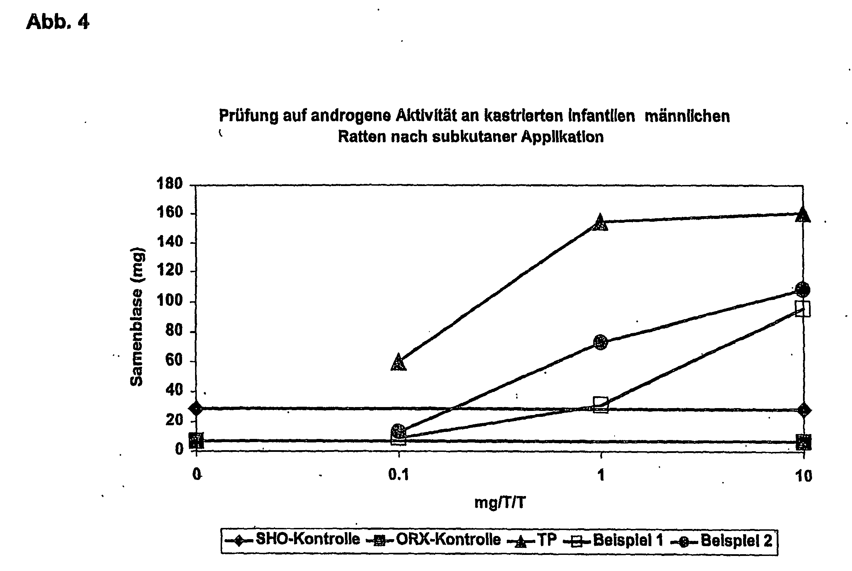

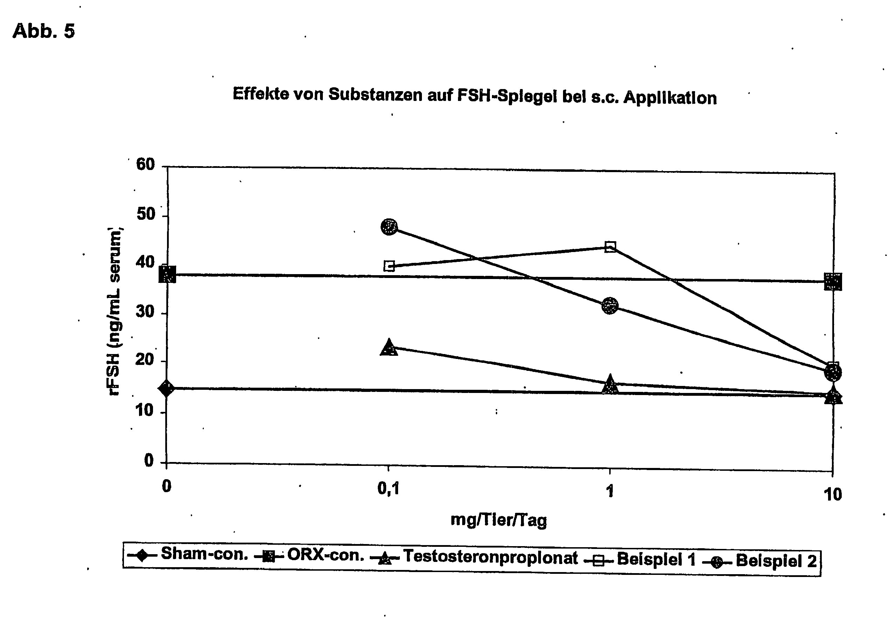

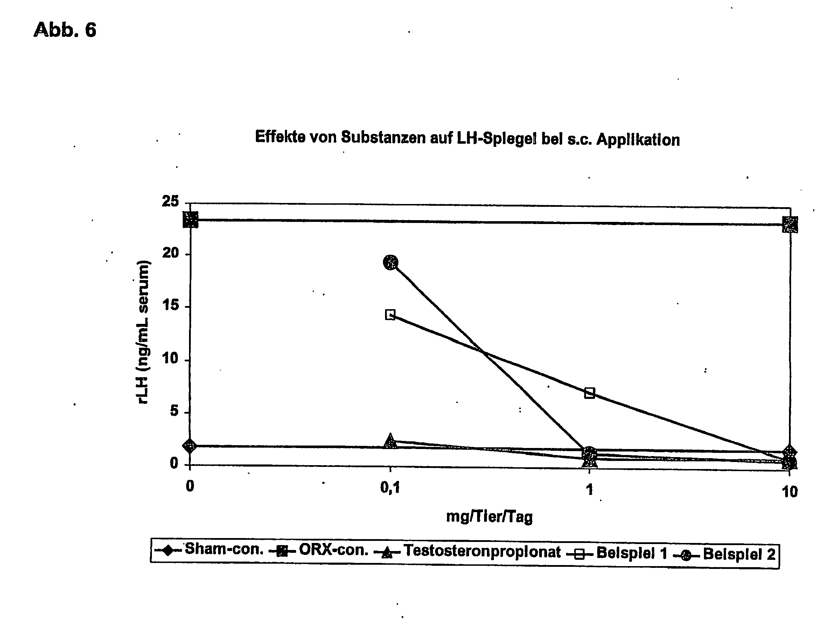

Männliche infantile Tiere (Stamm Mol : WIST, Tierzucht GmbH Schönwalde) im Gewicht von 40 - 50g wurden orchiektomiert (ORX). Eine Kontrollgruppe blieb unkastriert, wurde aber scheinoperiert (SHO). Die Kontrollgruppen ORX und SHO (n/Gruppe = 10 Tiere) erhielten das Vehikel der Prüfsubstanzen Benzylbenzoat/Ricinusöl appliziert.
Testosteronpropionat (Standard) und die Prüfsubstanzen wurden in den Dosen 0.1; 1.0 und 10 mg/Tier/Tag subkutan appliziert, Applikationsvolumen 0.2 ml/ Tier/ Tag.
24 Stunden nach der 7. Applikation wurden die Tiere authopsiert. Die Ergebnisse sind in den Abbildungen 3, 4, 5 und 6 dargestellt.
Substanzen mit einer AR-agonistischen und LH/FSH senkenden Wirkung in diesem Assay und PR-agonistischen und antagonistischen Eigenschaften, sind erfindungsgemäße Mesoprogestine.

### Ermittlung der estrogenen und osteoprotektiven Wirkung der Substanzen an adulten Ratten

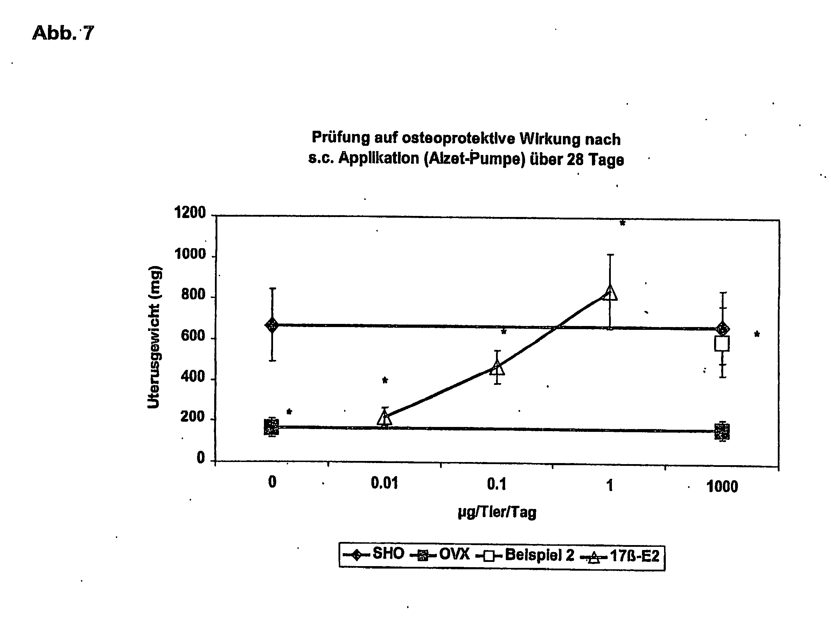

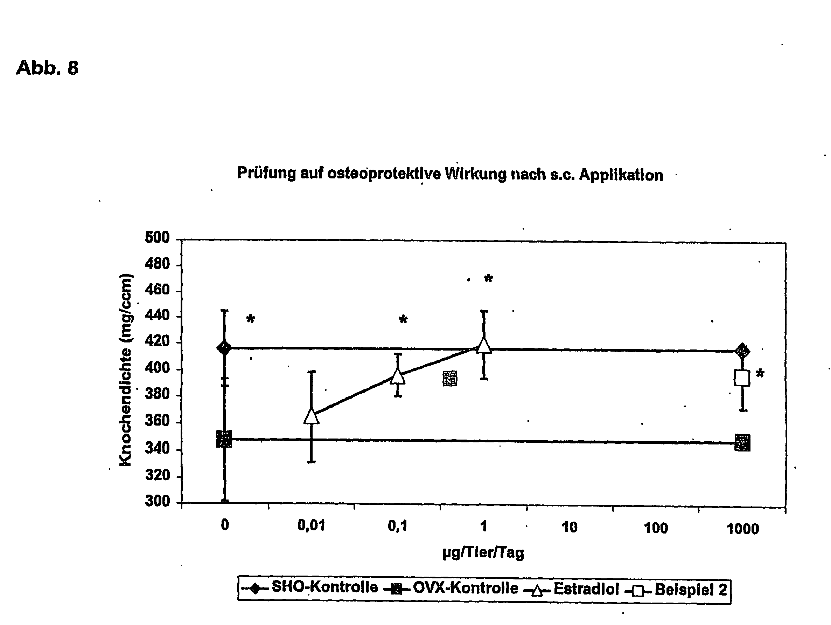

Die Prüfung auf estrogene und osteoprotektive Aktivität nach subkutaner Applikation über 28 Tage erfolgte an weiblichen, 6 Monate alten Ratten (Stamm Shoe : WIST, Tierzucht GmbH Schönwalde). Die Tiere wurden ovarektomiert und mit osmotischen Pumpen (Alzet) zur kontinuierlichen subkutanen Applikation der Prüfsubstanzen bestückt. Die Tiere wurden am Tag 29 authopsiert.
Zur Bestimmung der estrogenen Eigenschaften von Substanzen wurde das Uterusgewichts ermittelt. Die Knochendichte wurde mittels QCT (quantitativer Computertomographie) an der präparierten Tibia bestimmt. Die Ergebnisse sind in Abbildung 7 und 8 dargestellt.
Die Ergebnisse dieser Untersuchungen zeigen, dass erfindungsgemäße Substanzen per se osteoprotektiv wirken.

### Ermittlung der gestagenen Wirkung der Substanzen

Die Prüfung auf gestagene und antigestagene Aktivität erfolgte bei infantilen Kaninchen im McPhail - Test.

Das Prinzip stellt sich wie folgt dar: Im Zusammenwirken von Estrogenen und Progesteron wird das Endometrium für die Implantation der befruchteten Eizelle vorbereitet. Es kommt dabei zu ganz charakteristischen Veränderungen am Endometrium, die beim Kaninchen deutlich erkennbar sind. Oestrogene bewirken eine Proliferation. Die Wirkung von Gestagenen setzt erst ein, wenn das Endometrium proliferiert ist. Gestagene bewirken eine sogenannte transformatorische Umwandlung des Endometriums.

Infantile Kaninchen (New Zealand white, Züchter: Harlan Winkelmann) mit einem Gewicht von 700 - 900 g erhielten als Priming über 6 Tage (d1 - d6) einmal täglich 17β- Estradiolbenzoat (5 µg/Tier/Tag in 0,2 ml, s.c.). Am Versuchstag d7 begann die Applikation der Prüfsubstanzen sowie der Vergleichssubstanzen Progesteron und RU 486 täglich einmal über 7 Tage subkutan in Benzylbenzoat/Ricinusöl. Eine Kontrollgruppe erhielt täglich eine Vehikelapplikation (Benzylbenzoat/Ricinusöl).
Am Tag d14 wurde der Versuch beendet. Es erfolgte zunächst die Blutentnahme und anschließend die Authopsie.

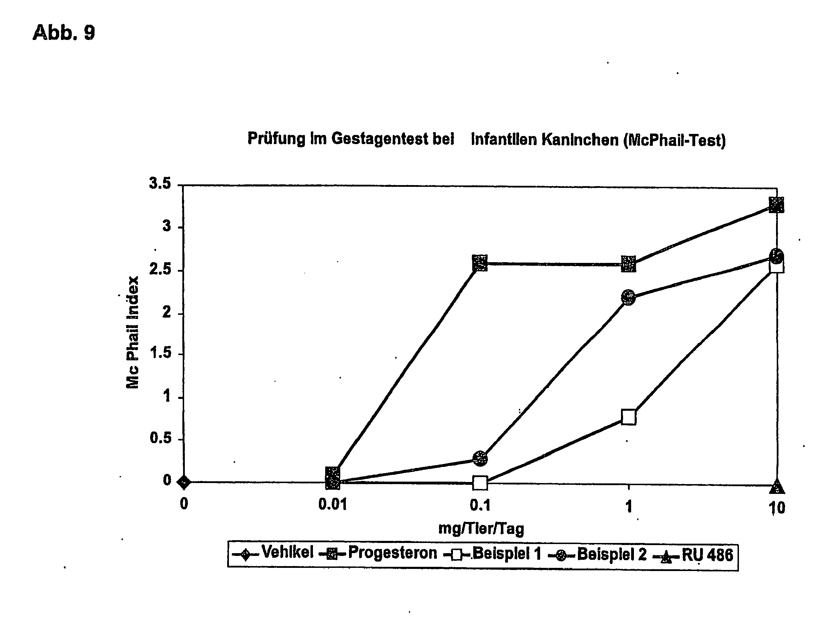

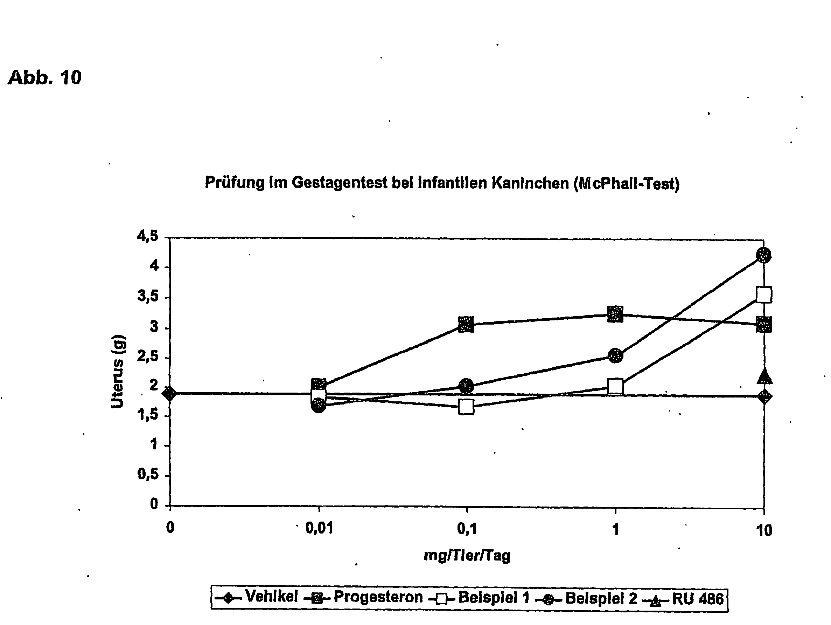

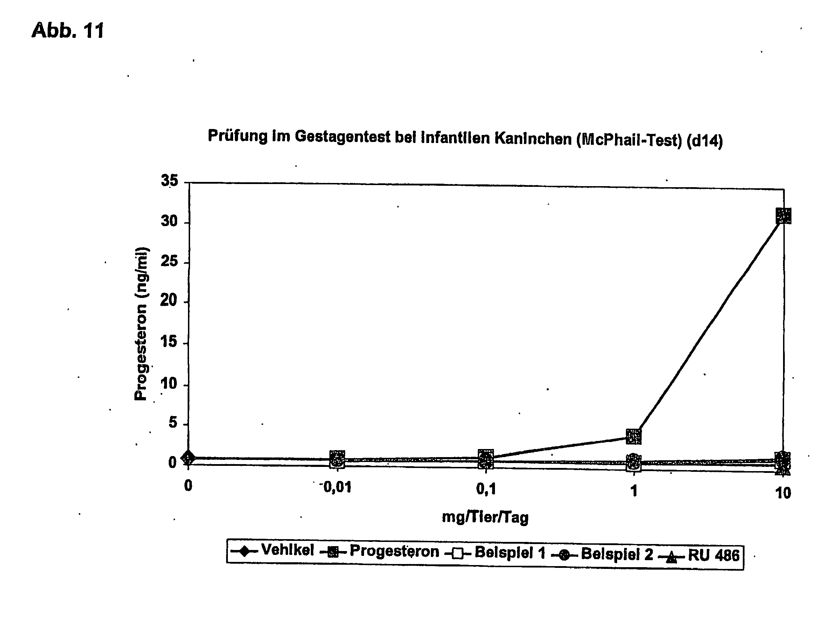

Die für die Gestagenwirkung typische Vermehrung sekretorischer Strukturen des Endometriums wird in den histologischen Präparaten nach der McPhail - Skala bewertet (Stadium. 1 - 4; 0 = keine Wirkung, 4 = volle Wirkung).
Die Ergebnisse sind in Abbildungen. 9, 10, 11 dargestellt.

Substanzen mit einer AR-agonistischen Wirkung, die die Wirkung von Progesteron in diesem Assay abschwächen, aber selbst submaximale Progesteron-analoge Effekte im Endometrium auslösen, sind erfindungsgemäße Mesoprogestine.

### Pharmazeutische Präparate und Indikationen

Die Verbindungen der allgemeinen Formel I stellen einen neuen Typ von Mesoprogestinen mit sowohl agonistischer Wirkung am Progesteronrezeptor als auch verbesserter antigonadotroper und milder androgener Wirksamkeit nach peroraler Applikation dar. Hierdurch werden bisher nicht mögliche Formen einer Hormonbehandlung erschlossen.
Die vorliegende Erfindung umfasst die neuen Substanzen als pharmazeutische Wirkstoffe, deren Herstellung, ihre therapeutische Anwendung und die pharmazeutischen Darreichungsformen, die die neuen Substanzen enthalten. Die hier beschriebenen neuen Mesoprogestine bzw. ihre pharmazeutisch annehmbaren Salze können sowohl ohne Estrogene als auch unter Zusatz von niedrig dosierten natürlichen Estrogenen wie Estradiol oder deren Ester zur Herstellung von Arzneimitteln dienen, die in der weiblichen Ferblitätskontrolle, in der weiblichen Hormonersatztherapie und für die Behandlung gynäkologischer Erkrankungen wie Endometriose, Uterus myomatoses, dysfunktionelle Blutungen und der Dysmenorrhoe eingesetzt werden können. Die Estrogene können hierbei auch in Form ihrer Sulfamate zum Einsatz kommen. Zur Herstellung und den besonderen pharmakologischen Eigenschaften der Sulfamate siehe J. Steroid Biochem. Molec. Biol, 55, 395 - 403 (1995); Exp. Opinion Invest. Drugs 7, 575 - 589 (1998).
Die neuen Progesteronrezeptormodulatoren eignen sich durch ihre hohe antiovulatorische Aktivität für eine vorzugsweise estrogenfreie oder estrogen-dosisreduzierte Kontrazeption der Frau. Die Blutungskontrolle wird im Gegensatz zu konventionellen Kontrazeptiva durch die Mesoprogestin/ Gestagenkomponente der erfindungsgemäßen Verbindungen übemommen. Ziel der Behandlung ist die Induktion einer Amenorrhoe. Die Estrogenkomponente hat in diesen Präparaten die Aufgabe ein Estrogendefizit zu vermeiden. Dadurch müssen die erfindungsgemäßen Verbindungen nicht mit EE kombiniert werden, sondern können vorzugsweise ohne Estrogene oder in Kombination mit geringen Dosen von natürlichen Estrogenen (z. B. Estradiol, dessen Ester, Estron, Estronsulfat, Estriol und Prodrugs dieser Estrogene) gegeben werden.

Durch den natürlichen Zyklus bzw. unter der hormonellen Kontrazeption mit einem kombinierten Kontrazeptivum (Estrogen- und Gestagenbehandlung) wird ein ständiges Auf und Ab des weiblichen Hormonzustandes provoziert. Damit kann eine deutliche Erhöhung des Risikos, an Brustkrebs, Ovarial- oder Endometriumskarzinom zu erkranken, verbunden sein [Coutinho, E.M. and Segal, S. *Is Menstruation Obsolete?,* Oxford University Press (1999)]. Klassische Gestagene stimulieren das Brustgewebe der Frau [Isaksson, E., von Schoultz, E., Odlind, V., et al. Effects of oral contraceptives on breast epithelial proliferation. *Breast Cancer Res Treat* 65:163-169 (2001)].
Das Spektrum hormonaler Eigenschaften der erfindungsgemäßen Substanzen hemmt dagegen die Proliferation in der Brust. Androgene haben einen deutlich hemmenden Effekt auf die Proliferation der Brustdrüse, entsprechend haben die erfindungsgemäßen Substanzen in dieser Hinsicht besondere Vorteile.
Die Verwendung der erfindungsgemäßen Substanzen zur Herstellung von Arzneimitteln zum Einsatz als Kontrazeptivum ermöglicht also ein völlig neues Konzept der Kontrazeption, bei der das Brustkrebsrisiko deutlich eingeschränkt wird.
Daneben lässt sich bei prämenopausalen Frauen der Zustand einer reversiblen Amenorrhoe ohne die negativen Anzeichen eines Estrogendefizits einstellen, da eine basale Estrogensekretion auch bei gehemmter Ovulation abhängig von der Dosis oft erhalten bleibt. Eine estrogenfreie oder estrogen-dosisreduzierte Kontrazeptionsmethode führt wiederum dazu, dass die bekannten Nebeneffekte wie thromboembolische Komplikationen deutlich reduziert werden können.
Die erfindungsgemäßen Substanzen bzw. ihre pharmazeutisch verträglichen Salze können ebenso als Einzelkomponente eingesetzt werden. Die Verwendung von niedrig dosierten, vorzugsweise natürlichen Estrogenen wie Estradiol und dessen Ester, führt zu einer Ruhigstellung der Ovarien und des Endometriums, was zu einer Reduzierung unerwünschter Proliferationserscheinungen in den genannten Geweben beiträgt.
Gegenüber konventionellen hormonalen HRT-Produkten ist das Fehlen jeglicher Blutungen ein wichtiges Merkmal. Daneben wird durch die erfindungsgemäßen Substanzen über deren androgene Aktivität der günstige Effekt ovarieller Androgene auf ZNS und Stoffwechselfunktionen substituiert. Anders als Gestagene stimulieren die erfindungsgemäßen Verbindungen das Drüsengewebe der Brust nicht.
Der besondere Vorteil der erfindungsgemäßen Verbindungen bezüglich der Behandlung von gynäkologischen Erkrankungen wie Endometriose, Uterus myomatoses, dysfunktionellen Blutungen und der Dysmenorrhoe liegt in deren erhöhter antifertiler Wirkung im Vergleich zu Verbindungen, die keine androgene Aktivität besitzen. Durch die erhöhte antiovulatorische Aktivität der erfindungsgemäßen Verbindungen werden Schwangerschaften unter der Behandlung von gynäkologischen Erkrankungen mit Mesoprogestinen ausgeschlossen.

Die erfindungsgemäßen Verbindungen der allgemeinen Formel I und deren Säureadditionssalze sind zur Herstellung pharmazeutischer Zusammensetzungen und Zubereitungen geeignet. Die pharmazeutischen Zusammensetzungen beziehungsweise Arzneimittel enthalten als Wirkstoff mindestens einen oder mehrere der erfindungsgemäßen Verbindungen der allgemeinen Formel I oder deren Säureadditionssalze, gegebenenfalls in Kombination mit anderen pharmakologisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.
Als derartige Träger- und Hilfsstoffe kommen zum Beispiel solche infrage, die in folgenden Literaturstellen als Hilfsstoffe für Pharmazie, Kosmetik und angrenzende Gebiete empfohlen bzw. angegeben sind: Ullmann's Enzyklopädie der technischen Chemie, 4, *1953*, 1-39; J. Pharm. Sciences, 52, 1963, 918 ff; H. v. Czetsch-Lindenwald, Hilfsstoffe für Pharmazie und angrenzende Gebiete; Pharm. Ind. 2, *1961*, 72 ff; Dr. H. P, Fiedler, Lexikon der Hilfsstoffe für Pharmazie, Kosmetik und angrenzende Gebiete Cantor KG. Aulendorf in Württemberg *1971*.

Die Formulierung der pharmazeutischen Präparate auf Basis der neuen Verbindungen erfolgt in an sich bekannter Weise, in dem man den Wirkstoff mit den in der Galenik gebräuchlichen Trägersubstanzen, Füllstoffen, Zerfallsbeeinflussern, Bindemitteln, Feuchthaltemitteln, Gleitmitteln, Absorptionsmitteln, Verdünnungsmitteln, Geschmackskonigentien, Färbemitteln usw. verarbeitet und in die gewünschte Applikationsform überführt. Dabei ist auf Remington's Pharmaceutical Science, 15^{th} ed. Mack Publishing Company, East Pennsylvania (1980) hinzuweisen.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder auch Depotformen.

Die erfindungsgemäßen Verbindungen der allgemeinen Formel bzw. die pharmazeutischen Zusammensetzungen enthaltend mindestens eine der erfindungsgemäßen Verbindungen werden bevorzugt oral appliziert.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmittein wie Maisstärke oder Alginsäure, Bindemitteln wie Stärke oder Gelatine, Gleitmitteln wie Magnesiumstearat oder Talk und/ oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titanoxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemäßen Verbindungen der allgemeinen Formel I können zusätzlich geschmacksverbessemde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten.
Die Verbindungen der allgemeinen Formel I enthaltende Kapseln können beispielsweise hergestellt werden, indem man die Verbindung der allgemeinen Formel I mit einem inerten Träger wie Milchzucker oder Sorbit mischt und in Gelatinekapseln einkapselt.

### Dosierung

Die zu verabreichende Menge der Verbindungen schwankt innerhalb eines weiten Bereiches und kann jede wirksame Menge abdecken.
In Abhängigkeit des zu erzielenden Effektes und der Art der Verabreichung kann die Menge der zu verabreichenden Verbindung einen Bereich von 0,01 bis 50 mg umfassen. Beim Menschen liegt eine empfohlene tägliche Dosis im Bereich von 0,05 bis 10 mg.
Geeignete Dosierungen für die erfindungsgemäßen Verbindungen betragen von 0,1 bis 10 mg.
Die erfindungsgemäßen Verbindungen werden kontinuierlich, vorzugsweise täglich bis einmal wöchentlich verabreicht.

Die erfindungsgemäßen Verbindungen sind geeignet für vaginale, intrauterine und subkutane Applikationen in geeigneten Trägersystemen (Elastomere).

Fallweise erlaubt diese Darreichungsform niedrigere Dosierungen als oben angegeben.

Die Erfindung umfasst ebenfalls die erfindungsgemäßen Verbindungen der allgemeinen Formel I als therapeutischen Wirkstoff zusammen mit pharmazeutisch verträglichen und annehmbaren Hilfsstoffen und/ oder Trägerstoffen.

Ebenfalls umfasst die Erfindung pharmazeutische Zusammensetzungen, die eine der pharmazeutisch aktiven, erfindungsgemäßen Verbindungen oder ein Gemisch von diesen oder ein pharmazeutisch verträgliches Salz sowie pharmazeutisch verträgliche Hilfsstoffe und Trägerstoffe enthalten.

Die erfindungsgemäßen Verbindungen der allgemeinen Formel I lassen sich wie nachstehend beschrieben herstellen:

Der Zugang zu den erfindungsgemäßen 11β-Benzaldoxim-estra-4,9-dien-Derivaten der allgemeinen Formel I erfolgt über das 3,3-Dimethoxy-estra-5(10),9(11)-dien-17-on [Pierdet A., Vignau M. FR 5183 (1966)], welches mit H₂O₂ in Gegenwart von Hexafluoraceton in das 3,3-Dimethoxy-5α,10α-epoxiestr-9(11)-en-17-on überführt wird [Costerousse G., Teutsch G., EP 5100 (1979); Teutsch G., Ojasoo T., Raynaud J.P.: J. Steroid Biochem. 31, ***1988*,** 549-565]. Die Einführung der 11β-Benzaldehydgruppierung erfolgt durch Grignard-Reaktion mit dem entsprechenden Brombenzaldehydacetal. Nach Funktionalisierung am C-17 wird das 11β-Benzaldehydacetal hydrolisiert und anschließend oximiert (siehe Formelschema 1):

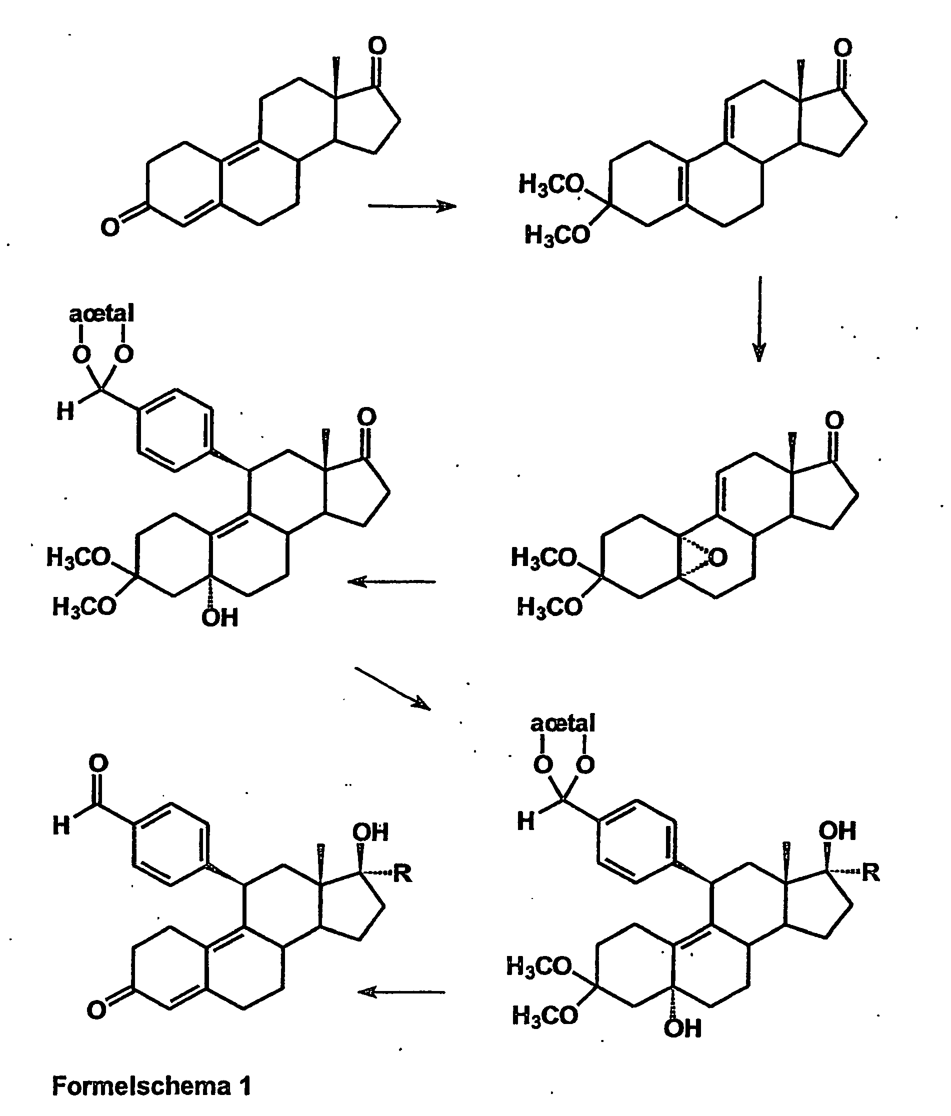

Die Einführung der Substituenten R₂ an C-4 erfolgt nach gängigen Methoden jeweils nach Einführung der Benzaldehydfunktion. Der Chlorsubstituent wird mit N-Chlorsuccinimid in Tetrahydrofuran, Brom analog mit N-Bromsuccinimid eingeführt. Die C-4-Substitution mit einer CF3-Gruppe wird wie von Fei et.al. (X.S. J. Fei et.al., Chem. Soc. Perkin Trans 1, ***1998*,** 1139-1142) beschrieben, durchgeführt.

Die nachfolgenden Beispiele dienen der näheren Erläuterung der Erfindung, ohne sie darauf einzuschränken:

### Beispiel 1

### 4-(17β-Hydroxy-3-oxoestra-4,9-dien-11β-yl)benzaldehyd-1(E)-oxim

115 mg 4-(17β-Hydroxy-3-oxoestra-4,9-dien-11β-yl)benzaldehyd werden in 2 ml Pyridin gelöst und portionsweise mit 23 mg Hydroxylaminhydrochlorid bei 23 °C umgesetzt. Nach 2 Stunden wird mit Eiswasser verdünnt, der Niederschlag wird abgesaugt, mit Wasser gewaschen und getrocknet. Man erhält 100 mg 4-(17β-Hydroxy-3-oxoestra-4,9-dien-11β-yl)benzaldehyd-1(E)-oxim, das durch präparative Schichtchromatographie an Kieselgel PF₂₅₄₊₃₆₆ mit Toluol /Aceton 4:1 gereinigt und aus Essigester umkristallisiert wird.
Schmelzpunkt: 150 bis 153 °C (Essigester)
α_{D} = +280 ° (CHCl₃)
¹H-NMR-Spektrum in CDCl₃ [δ, ppm] 0.42 (s, 3H, H-18), 3.68 (t, 1H, H-17α), 4.38 (d, J = 6.9 Hz, 1 H, H-11α), 5.79 (s, 1 H, H-4), 7.21 und 7.49 (m, 4 H, A,A', B,B'-System der Aromatenprotonen), 7.78 (s, 1 H, NOH), 8,11 (s, 1 H, CH=N-).

### Herstellung der Ausgangsverbindung

### Stufe 1

Zu einer Grignard-Lösung (hergestellt aus 1,6 g Magnesium und 17,7 g 4-Brombenzaldehydneopentylketal in 100 ml THF) gibt man bei - 20 °C 426 mg CuCI zu, rührt 10 min und tropft 5 g 3,3-Dimethoxy-5α,10α-epoxy-estr-9-en-17-on in 25 ml wasserfreiem THF hinzu. Bei 0°C rührt man 1,5 Stunden nach und zersetzt mit wässriger Ammoniumchlorid-Lösung. Nach Zugabe von Essigester werden die Phasen getrennt. Die organische Phase wird mit wässriger Ammoniumchlorid-Lösung und Wasser gewaschen, über Natriumsulfat getrocknet und unter vermindertem Druck eingedampft. Der helle Sirup wird an Kieselgel mit einem Toluol/Essigester-Gradienten gereinigt. Man erhält 3,24 g eines Rohproduktes. Durch Umkristallisation aus tert.-Butylmethylether / n-Hexan wird 3,3-Dimethoxy-11β-[4-(5,5-dimethyl-1,3-dioxan-2-yl)phenyl]-5α-hydroxy-estr-9-en-17-on als farblose Kristalle isoliert.
Schmelzpunkt: 194 bis 202°C (tert.-Butylmethylether/n-Hexan))
α_{D} = + 64° (CHCl₃)
¹H-NMR-Spektrum in CDCl₃ [δ, ppm] 0.46 (s, 3H, H-18), 0.80 und 1.31 (2s, je 3H, 2x CH₃) 3.20 und 3.33 (2s, je 3H, OCH₃), 3.63-3.79 (m, 4H, CH₂), 4.30 (d, J = 6,9 Hz, 1H, H-11α), 4.66 (s, 1H, OH), 5.35 (s, 1H, PhH ketal), 7.24 und 7.41 (2d, 4 H, A,A',B,B'-System der Aromatenprotonen).

### Stufe 2

2,1 g 3,3-Dimethoxy-11β-[4-(5,5-dimethyl-1,3-dioxan-2-yl)phenyl]-5α-hydroxy-estr-9-en-17-on werden in 25 ml Methanol / THF(1:1, V/V) gelöst und bei 23 °C mit 303 mg Natriumborhydrid reduziert. Nach 45 Minuten wird in Wasser eingerührt und die wässrige Phase mehrmals mit Methylenchlorid extrahiert. Die organische Phase wird getrocknet und unter vermindertem Druck verdampft. Das Rohprodukt [4-(3,3-Dimethoxy-5α17β-dihydroxy-estr-9-en-11β-yl)benz-aldehyd-neopentylketal] wird in 25 ml Aceton gelöst, mit 2 ml Wasser und 283 mg p-Toluolsulfonsäure bei Raumtemperatur 8 Stunden gerührt. Danach gießt man in Wasser ein und saugt den Niederschlag ab. Die Reinigung des Rohproduktes erfolgt durch Chromatographie mit einem Toluol/Aceton-Gradienten an Kieselgel. Man erhält 1,1 g 4-(17β-Hydroxy-3-oxoestra-4,9-dien-11β-yl)benz-aldehyd.
Schmelzpunkt: 197 bis 200°C (Aceton)
α_{D} = + 225° (CHCl₃)
¹H-NMR-Spektrum in CDCl3 [δ, ppm] 0.40 (s, 3H, H-18), 3.68 (t, 1H, J=9,0 Hz, H-17α) 4.44 (d, J=7,2 Hz, 1H, H-11α), 5.80 (s, 1 H, H-4), 7.38 (d, 2H, J = 8,1 Hz Aromatenprotonen) 7,81 (d, 2H, J = 6,6 Hz, Aromatenprotonen), 9.98 (s, 1H, CH=O).

### Beispiel 2

### 4-(17β-Hydroxy-17α-methyl-3-oxoestra-4,9-dien-11β-yl)benzaldehyd-1(E)-oxim

428 mg 4-(17β-Hydroxy-17α-methyl-3-oxoestra-4,9-dien-11β-yl)benzaldehyd werden nach Beispiel 1 in Pyridin mit 148 mg Hydroxylaminhydrochlorid bei 23 °C umgesetzt. Das Rohprodukt wird durch präparative Schichtchromatographie an Kieselgel PF₂₅₄₊₃₆₆ mit Toluol /Aceton 4:1 gereinigt und aus tert-Butylmethylether.umkristallisiert.
Schmelzpunkt: 154 bis 160 °C [tert.-Butylmethylether]
α_{D} = +224 ° (CHCl₃)
1 H-NMR-Spektrum in CDCl₃ [δ, ppm] 0.53 (s, 3H, H-18), 1.27 (s, 3H, 17α-CH₃), 4.42 (d, J = 6.9 Hz, 1 H, H-11α), 5.79 (s, 1 H, H-4), 7.22 und 7.50 (m, 4 H, A,A', B,B'-System der Aromatenprotonen), 7.79 (s, 1H, NOH), 8.11 (s, 1H, CH=N-).

### Herstellung der Ausgangsverbindung

Aus 3,3-Dimethoxy-5α,10α-epoxy-estr-9-en-17-on wird mit 4-Brombenzaldehyd-ethylenacetal via Grignard nach Beispiel 1 Stufe 1 die 11β-Benzaldehydethylenacetalgruppe eingeführt. Anschließend wird das Rohprodukt in THF zu einer Grignard-Lösung (hergestellt aus 2,43 g Magnesium und 6,4 ml Methyliodid in 25 ml tert.-Butylmethylether) zugetropft. Nach 2 Stunden wird die Grignard-Lösung mit wässriger Ammoniumchlorid-Lösung zersetzt und durch Extraktion wird nach üblicher Aufarbeitung das 4-(3,3-Dimethoxy-5α,17β-di-hydroxy-17α-methyl-estr-9-en-11β-yl)benzaldehyd-ethylenacetal isoliert.
Das Rohprodukt wird in 15 ml Aceton gelöst, mit 0,5 ml Wasser und 150 mg p-Toluolsulfonsäure versetzt und nach Beispiel 1 zu 4-(17β-Hydroxy-17α-methyl-3-oxoestra-4,9-dien-11β-yl)benzaldehyd hydrolisiert und durch Chromatographie gereinigt.
Schmelzpunkt: 160 bis 164 °C (Aceton)
α_{D} = + 211 ° (CHCl₃);
¹H-NMR-Spektrum in CDCl₃ [δ, ppm] 0.50 (s, 3H, H-18), 1.28 (s, 3H, 17α-CH₃), 4.48 (d, J = 7.2 Hz, 1 H, H-11α), 5.80 (s, 1 H, H-4), 7.38 (d, 2H, J = 8.1 Hz, Aromatenprotonen), 7.81 (d, 2H, J = 6.6 Hz, Aromatenprotonen), 9.98 (s, 1H, CH=O).

### Beispiel 3

### 4-(17β-Acetoxy-3-oxoestra-4,9-dien-11β-yl)benzaldehyd-1-(E)-oxim

450 mg 4-(17β-Acetoxy-3-oxoestra-4,9-dien-11β-yl)benzaldehyd werden in Pyridin mit 75 mg Hydroxylaminhydrochlorid nach Beispiel 1 umgesetzt. Das Rohprodukt wird durch präparative Schichtchromatographie an Kieselgel PF₂₅₄₊₃₆₆ gereinigt und aus Aceton umkristallisiert.
Schmelzpunkt: 130 bis 135 °C (Aceton)
α_{D} = +204° (CHCl₃)
¹H-NMR-Spektrum in CDCl₃ [δ, ppm] 0.47 (s, 3H, H-18), 2.03 (s, 3H, COCH₃), 4.35 (d, J = 6,9 Hz, 1 H, H-11α), 4.63 (t,1H, H-17α), 5.79 (s, 1 H, H-4), 7.21, 7.50 (2d, 4 H, A,A', B,B'-System der Aromatenprotonen), 7.64 (s, 1H, NOH), 8.11 (s, 1H, CH=N-)

### Herstellung der Ausgangsverbindung

437 mg 4-(17β-Hydroxy-3-oxoestra-4,9-dien-11β-yl)benzaldehyd werden mit 6 ml Acetanhydrid / Pyridin 1:1 bei Raumtemperatur innerhalb von 3 Stunden acetyliert. Durch Zugabe von Eiswasser wird ein Rohprodukt gefällt , das durch Umkristallisation aus Aceton gereinigt wird. Man erhält 226 mg 4-(17β-Acetoxy-3-oxoestra-4,9-dien-11β-yl)benzaldehyd.
Schmelzpunkt.: 188 bis 191 °C (Aceton)
α_{D} = + 202° (CHCl₃);
¹H-NMR-Spektrum in CDCl3 [δ, ppm]: 0.44 (s, 3H, H-18), 2.04 (s, 3H, COCH₃), 4.40 (d, J=6,9 Hz, 1H, H-11α), 4:63 (t, 1 H, J=8,1 Hz, H-17α), 5.80 (s, 1 H, H-4), 7,37 (d, 2H, J = 8,1 Hz, Aromatenprotonen), 7.80 (d, 2H, J = 8,1 Hz, Aromatenprotonen), 9.98 (s, 1 H, CH=O).

### Beispiel 4

### 4-[17β-(N-Ethylamino)carbonyloxy-3-oxoestra-4,9-dien-11βyl]benzaldehyd-1(E)-oxim

500 mg 4-[17β-(N-Ethylamino)carbonyloxy-3-oxoestra-4,9-dien-11β-yl]benz-aldehyd werden mit Hydroxylaminhydrochlorid in Pyridin nach Beispiel 1 umgesetzt und durch Chromatographie gereinigt. Man erhält 318 mg als farblosen Schaum aus Aceton.
α_{D} = + 218° (CHCl₃)
¹H-NMR-Spektrum in CDCl₃ [δ, ppm]: 0.43 (s, 3H, H-18), 1.15 (t, 3H, J=7.2 Hz,CH₂CH₃), 3.23 (t, J=6.6 Hz, 17α H), 4.35 (d, 1H, J = 7.2 Hz, H-11α), 4.6 (m, NHCH₂), 5.79 (s, 1H, H-4), 7.21 (d, 2H, J = 7.8 Hz, Aromatenprotonen), 7.50 (d, 2H, J = 7.8 Hz, Aromatenprotonen), 7.9 (s, 1 H, NOH), 8.11 (s, 1 H, CH=N).

### Herstellung der Ausgangsverbindung

870 mg 4-(17β-Hydroxy-3-oxoestra-4,9-dien-11β-yl)benzaldehyd (Beispiel 1) werden in 30 ml Toluol mit 2,4 ml Ethylisocyanat 8h unter Rückfluss erhitzt. Man kühlt ab, setzt 6 ml wässrigen NH₃ zu, rührt 1 h bei Raumtemperatur und extrahiert mehrmals mit CH₂Cl₂. Die organische Phase wird neutral gewaschen, über Na₂SO₄ getrocknet, und unter Vakuum verdampft der Rückstand wird durch Chromatographie gereinigt. Man erhält 530 mg 4-[17β-(N-Ethylamino)-carbonyloxy-3-oxoestra-4,9-dien-11βyl]benzaldehyd als hellen Schaum, der direkt in die Oximierung eingesetzt wird.
¹H-NMR-Spektrum in CDCl₃ [δ, ppm]: 0.41 (s, 3H, H-18), 1.14, 1.18 (2t, 3H, J=7.2, 7.8 Hz, CH₂CH₃), 3.22 (t, J=6.6 Hz, 17α H), 4.41 (d, 1 H, J = 7.8 Hz, H-11 α), 4.59 (t J=7.8 Hz, , NHCH₂), 5.80 (s, 1H, H-4), 7.37 (d, 2H, J = 8.1 Hz, Aromatenprotonen), 7.81 (d, 2H, J = 8.1 Hz, Aromatenprotonen), 9.98 (s, 1H, CH=O).

### Beispiel 5

### 4-(17β-Methoxy-3-oxoestra-4,9-dien-11β-yl)benzaldehyd-1(E)-oxim

Analog Beispiel 1 aus 4-(17β-Methoxy-3-oxoestra-4,9-dien-11β-yl)benzaldehyd und Hydroxylaminhydrochlorid in Pyridin.
Schmelzpunkt.: 111 bis 113°C ( Aceton )
α_{D} = + 262° (CHCl₃);
¹H-NMR-Spektrum in CDCl3 [δ, ppm]: 0.43 (s, 3H, H-18), 3.25 (t, 1H, J=8.0 Hz, 17α-H), 3.34 (s, 3H, OCH₃), 4.35 (d, J=7.2 Hz, 1 H, H-11α), 5.78 (s, 1H, H-4), 7.20 (d, 2H, J = 8.8 Hz Aromatenprotonen), 7.48 (d, 2H, J = 8.4 Hz, Aromatenprotonen), 8.09 (s, 1 H, CH=N-).

### Herstellung der Ausgangsverbindung

Aus 3,3-Dimethoxy-5α,10α-epoxy-estr-9-en-17-on wird mit 4-Brombenzaldehyddimethylacetal via Grignard nach Beispiel 1 Stufe 1 eine 11β-Benzaldehyddimethylacetal-Gruppierung eingeführt und anschließend gemäß Beispiel 1 die 17-Ketofunktion mit Natriumborhydrid reduziert.
5,26 g 4-(3,3-Dimethoxy-5α,17β-dihydroxy-estr-9-en-11β-yl)benzaldehyd-di-methylacetal werden in 50 ml Toluol gelöst, mit 3,37 g Kalium tert.-butanolat und anschließend mit 1,9 ml Methyliodid versetzt. Nach 4 h wird mit Wasser verdünnt und die organische Phase mit wässriger Ammoniumchloridlösung und Wasser neutral gewaschen, über Natriumsulfat getrocknet und unter Vakuum eingeengt.
Das gelbe Öl von 4-(5α-Hydroxy-3,3,17β-trimethoxy-estr-9-en-11β-yl)benz-aldehyd-dimethylacetal wird in 50 ml Aceton gelöst, mit 650 mg p-Toluolsulfonsäure versetzt und 12 h bei Raumtemperatur gerührt. Man gießt in 0,4 l Eiswasser, wobei 4-(17β-Methoxy-3-oxoestra-4,9-dien-11β-yl)benzaldehyd als ein farbloses Produkt ausfällt, das abgesaugt und neutral gewaschen wird. Nach Reinigung durch Säulenchromatographie an Kieselgel wird ein Rohprodukt erhalten, das aus Aceton umkristallisiert wird.
Schmelzpunkt: 133 bis 135°C ( Aceton )
α_{D} = + 244° (CHCl₃);
¹H-NMR-Spektrum in CDCl₃ [δ, ppm]: 0.40 (s, 3H, H-18), 3.25 (t, 1H, J=8.0 Hz, 17α-H), 3.33 (s, 3H, OCH₃), 4.41 (d, J=7.2 Hz, 1 H, H-11α), 5.78 (s, 1 H, H-4), 7.37 (d, 2H, J = 8.0 Hz Aromateriprotonen), 7.79 (d, 2H, J = 8.4 Hz, Aromatenprotonen), 9.96 (s, 1 H, CH=O).

### Beispiel 6

### 4-(4 '-Brom-17β-hydroxy-17α-methyl-3-oxoestra-4,9-dien-11β-yl)benzaldehyd-1(E)-oxim

Analog Beispiel 1 aus 4-(4'-Brom-17β-hydroxy-17α-methyl-3-oxoestra-4,9-dien-11 β-yl)benzaldehyd und Hydroxylaminhydrochlorid in Pyridin.
Schmelzpunkt: 157 °C (Zersetzung, Ether)
α_{D} = + 175 ° (CHCl₃)
¹H-NMR-Spektrum in CDCl₃ [δ, ppm] 0.53 (s, 3H, H-18), 1.25 (s, 3H, 17α-CH₃), 4.41 (d, J = 7.2 Hz, 1 H, H-11α), 7.17 und 7.47 (2d, 4H, J= 8.0 Hz, A,A', B,B'-System der Aromatenprotonen), 8.08 (s, 1 H, CH=N-).

### Herstellung der Ausgangsverbindung

781 mg 4-(17β-Hydroxy-17α-methyl-3-oxoestra-4,9-dien-11β-yl)benzaldehyd werden in 25 ml Tetrahydrofuran gelöst und mit 356 mg N-Bromsuccinimid versetzt . Das Gemisch wird 2 h bei Raumtemperatur gerührt, danach in 200 ml Eiswasser gegossen. Der Niederschlag wird abfiltriert, neutral gewaschen und getrocknet. Nach Reinigung mittels präparativer Schichtchromatographie werden 475mg 4-(4'-Brom-17β-hydroxy-17α-methyl-3-oxtiestra-4,9-dien-11β-yl)benzaldehyd als Schaum erhalten, der direkt in die Oximierung analog Beispiel 1 mit Hydroxylammoniumhydrochlorid in Pyridin eingesetzt wird.
1H-NMR-Spektrum in CDCl₃ [δ, ppm]: 0.52 (s, 3H, H-18), 1.27 (s, 3H, 17α-CH₃), 3,21 (2t, 1 H, OH), 4.41 (d, J = 7.2 Hz, 1 H, H-11α), 7.34 und 7.79 (2d, 4 H, J= 8.0 Hz, A,A', B,B'-System der Aromatenprotonen), 9.96 (s, 1 H, CH=O).

### Beispiel 7

### 4-(4'-Brom-17β-methoxy-3-oxoestra-4,9-dien-11β-yl)benzaldehyd-1(E)-oxim

Herstellung analog Beispiel 1 aus 4-(4'-Brom-17β-methoxy-3-oxoestra-4,9-dien-11 β-yl)benzaldehyd mit Hydroxylaminhydrochlorid in Pyridin.
Schmelzpunkt: 145 ° Zers. (tert. Butylmethylether)
α_{D} = +198 ° (CHCl₃)
1 H-NMR-Spektrum in CDCl₃ [δ, ppm]: 0.44 (s, 3H, H-18), 3.26 (t, 1 H, H-17α), 3.34 (s, 3H , OCH₃), 4.34 (d, J = 7.6 Hz, 1 H, H-11α), 7.17 und 7.47 (2d, 4 H, J=8 Hz, A,A', B,B'-System der Aromatenprotonen), 8,08 (s, 2H, =NOH und CH=N-).

### Herstellung der Ausgangsverbindung

Analog Beispiel 6 aus 4-(17β-Methoxy-3-oxoestra-4,9-dien-11β-yl)benzaldehyd mit N-Bromsuccinimid in THF.
blaßgelber Schaum, der direkt in die Oximierung eingesetzt wird.
¹H-NMR-Spektrum in CDCl₃ [δ, ppm] 0.42 (s, 3H, H-18), 3.26 (t, 1 H, H-17α), 3.33 (s, 3H, OCH₃), 4.40 (d, J = 7.2 Hz, 1H, H-11α), 7.34 und 7.79 (2d, 4 H, J=8 Hz, A,A',B,B'-System der Aromatenprotonen), 9.96 (s, 1 H, CH=O).

### Beispiel 8

### 4-(4'-Brom-17β-hydroxy-17α-trifluormethyl-3-oxoestra-4,9-dien-11β-yl)benzaldehyd-1(E)-oxim

Herstellung analog Beispiel 1 aus 4-(4'-Brom-17β-hydroxy-17α-trifluormethyl-3-oxoestra-4,9-dien-11β-yl)benzaldehyd und Hydroxylaminhydrochlorid in Pyridin. Schmelzpunkt: 198 bis 203 °C (Ether, n-Hexan)
α_{D} = + 154 ° (CHCl₃)
¹H-NMR-Spektrum in CDCl₃ [δ, ppm]: 0.60 (s, 3H, H-18), 3.22 (2t, 1 H, OH), 4.44 (d, J = 7.2 Hz, 1H, H-11α), 7.17 und 7.48 (2d, 4H, J= 8.0 und 8.8 Hz, A,A', B,B'-System der Aromatenprotonen), 7.88 (s, 1 H, NOH), 8.09 (s, 1 H, CH=N-).

### Herstellung der Ausgangsverbindungen

### 4-(4'-Brom-17β-hydroxy-17α-trifluormethyl-3-oxoestra-4,9-dien-11β-yl)benzaldehyd

Analog Beispiel 6 aus 4-(17β-Hydroxy-17α-trifluonnethyl-3-oxoestra-4,9-dien-11 β-yl)benzaldehyd mit N-Bromsuccinimid in THF.
hellgelber Schaum, der direkt in die Oximierung eingesetzt wird.
¹H-NMR-Spektrum in CDCl₃ [δ, ppm]: 0.59 (s, 3H, H-18), 3.22 (2t, 1 H, OH), 4.50 (d, J=7.6 Hz, 1H, H-11α), 7.35 und 7.80 (2d, 4H, J= 8.4 Hz, A,A', B,B'-System der Aromatenprotonen), 9.96 (s, 1 H, CH=O).

### 4-[17β-Hydroxy-17α-(1,1,1-trifluormethyl)-3-oxoestra-4,9-dien-11β-yl]benz-aldehyd

1g 3,3-Dimethoxy-11β-{[4-(1,1-ethylendioxy)methyl)phenyl}5α-hydroxy-estr-9-en-17-on wird in 30 ml abs. THF gelöst, mit 1,0 g Molsieb 3A versetzt und 30 Minuten unter Argon gerührt. Man kühlt auf 0°C ab, tropft 1,5 ml Trifluormethyltrimethylsilan zu, rührt 10 h Minuten nach und gibt dann 1 g Tetrabutylammoniumfluorid zu. Nach 10 bei 5 °C wird die Reaktionslösung durch Zugabe von 10 ml 1 n HCl zersetzt. Man lässt auf Raumtemperatur kommen, gibt jeweils 100 ml Wasser und Essigester zu, trennt die Phasen, wäscht die organische Phase neutral, trocknet über Natriumsulfat, filtriert die organische Phase ab und engt unter Vakuum ein. Nach Zugabe von Aceton verbleiben 1,05 g gelbe Kristalle. Umkristallisation aus Aceton und Behandlung mit tert.-Butylmethylether ergibt 480 mg 4-[17β-Hydroxy-17α-(1,1,1-trifluormethyl)-3-oxoestra-4,9-dien-11 β-yl]benzaldehyd.
Schmp.: 284 bis 292 °C (Aceton)
α_{D} = + 221 ° (CHCl₃)
¹H-NMR-Spektrum in CDCl₃: [δ, ppm ,]: 0.58 (s, 3H, H-18), 4.51 (d, 1 H, J = 7.1 Hz, H-11α), 5.81 (s, 1 H, H-4), 7.38 (d, 2H, J = 8.3 Hz, Aromatenprotonen), 7.81 (d, 2H, J = 8.3 Hz), 9.97 (s, 1 H, CH=O).

### Beispiel 9

### 4-[17β-Hydroxy-17α-methyl-3-oxoestra-4,9-dien-11β-yl]benzaldehyd-1-(E)-[O-(ethylamino)-carbonyl]oxim

Analog Beispiel 4 aus 4-(17β-Hydroxy-17α-methyl-3-oxoestra-4,9-dien-11β-yl)-benzaldehyd-1(E)-oxim mit Ethylisocyanat in Toluol.
Schmelzpunkt.: 178 bis 183°C (Aceton/n-Hexan)
α_{D} = + 264° (CHCl₃);
¹H-NMR-Spektrum in CDCl₃ [δ, ppm]: 0.52 (s, 3H, H-18), 1.24 (t, 3H, CH₂CH₃), 1.62 (s, 3H, CH₃), 3.38 (m, 2H, CH₂), 4.45 (d, J=7.2 Hz, 1 H, H-11α), 5.80 (s, 1H, H-4), 6.24 (t, 1H, NH), 7.28 (d, 2H, J = 8.4 Hz, Aromatenprotonen), 7.59 (d, 2H, J = 8.1 Hz, Aromatenprotonen), 8.30 (s, 1 H, CH=N-).

### Beispiel 10

### 4-(17α-Brommethyl-17β-hydroxy-3-oxoestra-4,9-dien-11β-yl)benzaldehyd-1(E)-oxim

Herstellung analog Beispiel 1 aus 4-(17α-Brommethyl-17β-hydroxy-3-oxoestra-4,9-dien-11β-yl)benzaldehyd mit Hydroxylamin in Pyridin.
Schmelzpunkt: 107 bis 109°C (Aceton)
α_{D} = +161 ° (CHCl₃)
¹H-NMR-Spektrum in CDCl₃ [δ, ppm]: 0.63 (s, 3H, H-18), 3.60 (d, 2H, J= 10,4 Hz, CH₂), 3.79 (d, 2H, J= 9.6 Hz, CH₂), 4.42 (d, J = 7.2 Hz, 1 H, H-11α), 5.80 (s, 1H, H-4), 7.18 und 7.48 (2d, 4 H, J= 8.4 Hz, A,A', B,B'-System der Aromatenprotonen), 8.09 (s, 1 H, CH=N-), 8.27 (s, 1 H, NOH).
LC/MS: 484.0 (M⁺ + H)

### Herstellung der Ausgangsverbindung

5 mmol 4-(3,3-Dimethoxy-5α-hydroxy-17-oxoestr-9-en-11β-yl)benzaldehyd-ethylenacetal werden nach Corey, E.J. Chaykowsky, J. (J. Am.Chem.Soc. **1962,** 84, 3782) in DMF mit 10 mmoi Trimethylsulfoniumiodid und 10 mmol Kalium-tert.butanolat bei Raumtemperatur innerhalb von 3 Stunden zum Spiroepoxid umgesetzt. 1,98 g 4-(3,3-Dimethoxy-5α-hydroxy-17(S)-spiroepoxy estr-9-en-11β-yl)benzaldehyd-ethylenacetal werden in 30 ml Dimethylformamid gelöst und auf ca. 0°C abgekühlt . Nach Zugabe von 5 ml Bromwasserstoffsäure wird noch 10 min bei 0°C gerührt und dann lässt man den Ansatz auf Raumtemperatur kommen. Nach 1h wird das Reaktionsgemisch in 400 ml wässrige Natriumbicarbonatlösung eingerührt, wobei ein blassgelber Niederschlag ausfällt, der abgesaugt, neutral gewaschen und getrocknet wird. Man erhält 1,69 g 4-(17α-Brommethyl-17β-hydroxy-3-oxoestra-4,9-dien-11β-yl)benzaldehyd, der durch Chromatographie gereinigt wird. Man erhält einen farblosen Schaum, der direkt in die Oximierung eingesetzt wird.
LC/MS: 469.0 (M⁺ +H)
¹H-NMR-Spektrum in CDCl₃ [δ, ppm]: 0.61(s, 3H, H-18), 2.33 (s, 1 H, OH), 3.60 und 3.78 (2d, 2H, J=10,4 Hz und J=10.0 Hz, CH₂), 4.49 (d, J = 6.8 Hz, 1 H, H-11 α), 5.80 (s, 1H, H-4), 7.36 und 7.80 (2d, 4H, J=8.0 Hz, A,A', B,B'-System der Aromatenprotonen), 9.96 (s, 1 H, CH=O).

### Beispiel 11

### 4-[17β-Hydroxy-3-oxoestra-4,9-dien-11β-yl]benzaldehyd-1-(E)-[O-(ethylamino)-carbonyl]oxim

Analog Beispiel 4 aus 4-(17β-Hydroxy-3-oxoestra-4,9-dien-11β-yl)benzaldehyd-1(E)-oxim mit 3 Äquivalenten Ethylisocyanat in Toluol/ Aceton-Gemisch (3:1). Schmelzpunkt: ab 127°C Zers. (Essigester)
α_{D} = + 263° (CHCl₃)
¹H-NMR-Spektrum in CDCl3 [δ, ppm]: 0.42 (s, 3H, H-18), 1.24 (t, 3H, CH₂CH₃), 1.74 (s, 1H, OH), 3.68 (t, 1H, H-17α), 4.11 (q, 2H, ΣJ= 21.6 Hz, CH₂Me), 4.40 (d, J=6.8 Hz, 1 H, H-11α), 5.78 (s, 1 H, H-4), 6.24 (t, 1 H, NH), 7.27 (d, 2H, J = 8.4 Hz, Aromatenprotonen), 7.59 (d, 2H, J = 8.1 Hz, Aromatenprotonen), 8.29 (s, 1 H, CH=N-).

### Beispiel 12

### 4-(17β-Hydroxy-3-oxoestra-4,9,15-trien-11β-yl)benzaldehyd-1(E)-oxim

Analog Beispiel 1 aus dem 4-(17β-Hydroxy-3-oxoestra-4,9,15-trien-11β-yl)benz-aldehyd mit Hydroxylaminhydrochlorid in Pyridin.
Schmelzpunkt: 229 bis 231 °C (Aceton)
α_{D} = + 298° (CHCl₃)
¹H-NMR-Spektrum in CDCl₃ [δ, ppm]: 0.54 (s, 3H, H-18), 1.63 (s, 1 H, OH), 4.36 (s, 1 H, 17α), 4.43 (d, J = 8.0 Hz, 1 H, H-11α), 5.72 (d, 1 H, J = 6.4 Hz, olefin. Proton), 5.79 (s. 1 H, H-4), 5.93 (d, 1 H, J 6.4 Hz, olefin. Proton), 7.19 und 7.48 (2d, 4H, J= 8.4 und 8.8 Hz, A,A', B,B'-System der Aromatenprotonen), 7.61 (s, 1 H, NOH), 8.09 (s, 1 H, CH=N-).

### Herstellung der Ausgangsverbindung

4,8 g 3,3-Dimethoxy-11β-{[4-(1,1-ethylendioxy)methyl]phenyl}5α-hydroxy-estr-9-en-17-on werden in 180 ml THF gelöst. Bei -70 °C werden unter Argonschutz nacheinander 15 ml einer 2M Lösung von Lithiumdiisopropylamid in THF/ Heptan/ Ethylbenzen und 5,1 ml Trimethylchlorsilan zugetropft. Man lässt auf Raumtemperatur kommen, rührt in wässrige Natriumbicarbonat-Lösung ein, extrahiert mit Essigester und arbeitet wie üblich auf. Das 4-(3,3-Dimethoxy-5α-hydroxy-17-trimethylsilyioxy-estr-9,15-dien-11β-yl)benzaldehyd-ethylenacetal wird als gelber Schaum isoliert. 6,1 g des Rohproduktes werden in 50 ml Acetonitril gelöst, mit 2,47 g Palladium(II)-acetat versetzt, 4 Stunden bei Raumtemperatur gerührt, über Kieselgur filtriert, eingeengt, chromatographiert und aus tert.-Butylmethylether umkristallisiert.
961 mg des dabei erhaltenen 4-(3,3-Dimethoxy-5α-hydroxy-17-oxoestra-9,15-dien-11β-yl)benzaldehyd-ethylenacetal werden in 5 ml Methanol gelöst, auf - 10 °C abgekühlt, mit 30 mg Natriumbicarbonat und anschließend mit 745 mg CeCl₃ x 7 H2O und 348 mg Natriumborhydrid in 20 ml Methanol versetzt. Man rührt nach 20 Minuten in Eiswasser ein, saugt das 4-(5α,17β-Dihydroxy-3,3-dimethoxy-estra-9,15-dien-11β-yl)benzaldehyd-ethylenacetal ab, wäscht mit Wasser nach und löst die Substanz mit Aceton. Nach Zugabe von 1 ml Wasser und 150 mg p-Toluolsulfonsäure werden die Schutzgruppen abgespalten und nach 2 Stunden wird in Eiswasser eingerührt. Man saugt ab, trocknet und kristallisiert nach der Chromatographie den 4-(17β-Hydroxy-3-oxoestra-4,9,15-trien-11β-yl)benzaldehyd aus Aceton um.
Schmelzpunkt:180 bis 183 °C (Aceton)
α_{D} = + 162 ° (CHCl₃)
¹H-NMR-Spektrum in CDCl₃ [δ, ppm]: 0.53 (s, 3H, H-18), 1.63 (s, 1 H, OH), 4.37 (s, 1 H, H -17α), 4.50 (d, 1 H J= 7.6 Hz, H-11α), 5.72 (d, 1 H, olefin. Proton), 5.80 (s, 1H, H-4), 5.93 (d, 1 H, J= 7.67 Hz, olefin. Proton), 7.36 und 7.80 (2d, 4H, J=8.0 Hz und 8,8 Hz, A,A', B,B'-System der Aromatenprotonen), 9.96 (s, 1H, CH=O).

### Beispiel 13

### 4-(17β-Acetoxy-4'-brom-3-oxoestra-4,9-dien-11β-yl)benzaldehyd-1-(E)oxim

528 mg 4-(4'-Brom-17β-hydroxy-3-oxoestra-4,9-dien-11β-yl)benzaldehyd werden nach Beispiel 3 acetyliert und anschließend nach Beispiel 1 oximiert. Schmelzpunkt: 158 - 161°C (Zersetzung, Aceton)
α_{D} = + 155 ° (CHCl₃)
¹H-NMR-Spektrum in CDCl₃ [δ, ppm]: 0.48 (s, 3H, H-18), 2.04 (s, 3H, COCH₃), 4.35 (d, J = 7.6 Hz, 1 H, H-11α), 4.62 (t, 1 H J= 7.6 Hz, H17α), 7.16 und 7.49 (2d, 4H, J= 8.0 und 8,8 Hz, A,A', B,B'-System der Aromatenprotonen), 7.92 (s, 1H, NOH), 8,10 (s, 1 H, CH=N-).

### Beispiel 14

### 4-(17β-Acetoxy-4'-brom-3-oxoestra-4,9-dien-11β-yl)benzaldehyd-1-(E)-O-acetyloxim

100 mg 4-(17β-Acetoxy-4'-brom-3-oxoestra-4,9-dien-11β-yl)benzaldehyd-1[E]-oxim werden nach Beispiel 3 acetyliert.
Schmelzpunkt: 114 bis 118 °C (Diethylether/n-Hexan)
α_{D} = + 147 ° (CHCl₃)
¹H-NMR-Spektrum in CDCl₃ [δ, ppm]: 0.45 (s, 3H, H-18), 2.03 und 2.23 (2s, 2 x 3H, 2x COCH₃), 4.37 (d, J = 7.2 Hz, 1 H, H-11α), 4.62 (t,1H, J=8.4 Hz, H-17α), 7.22 und 7.65 (2d, 4H, J= 8.0 Hz, A,A', B,B'-System der Aromatenprotonen), 8.32 (s, 1 H, CH=N-).

### Beispiel 15

### 4-(17β-Ethoxy-3-oxoestra-4,9-dien-11β-yl)benzaldehyd-1(E)-oxim

Aus 4-(17β-Ethoxy-3-oxoestra-4,9-dien-11β-yl)benzaldehyd mit Hydroxylaminhydrochloid in Pyridin nach Beispiel 1
Schmelzpunkt: 100 bis 103°C ( Aceton )α_{D} = + 256° (CHCl₃)
¹H-NMR-Spektrum in CDCl3 [δ, ppm]: 0.44 (s, 3H, H-18), 1.71 (t, 3H, CH₂CH₃) 3.33 (t, 1H, J=8.0 Hz,17α-H), 3.5 (m, 2H, CH₂CH₃), 4.34 (d, J=7.6 Hz, 1 H, H-11 α), 5.77 (s, 1 H, H-4), 7.20 (d, 2H, J = 8.0 Hz Aromatenprotonen), 7.48 (d, 2H, J = 8.4 Hz, Aromatenprotonen), 7.69 (s, 1 H NOH), 8.10 (s, 1H, CH=N-).

### Herstellung der Ausgangsverbindung

Analog Beispiel 5 aus 4-(3,3-Dimethoxy-5α,17β-dihydroxy-estr-9-en-11β-yl)-benzaldehyd-neopentylacetal und Ethylbromid und Kalium-tert.-butanolat in THF zum 4-(3,3-Dimethoxy-17β-ethoxy-5α-hydroxy-estr-9-en-11β-yl)benzaldehydneopentylacetal.
Schmelzpunkt: 161 bis 168 °C (Methanol)
α_{D} = + 15° (CHCl₃)
¹H-NMR-Spektrum in CDCl3 [δ, ppm]: 0.36 (s, 3H, H-18), 0.86 und 1.03 (2s, je 3H, 3-acetal CH₃), 1.14 (t, 3H, CH₂CH₃) 3.26 (t, 1H, J=8.0 Hz,17α-H), 3.3 (2s, 6H, 2x OCH₃), 3.48 bis 3.57 (m, 6H, 3 x CH₂), 4.22 (d, 1 H, H-11α), 4.39 [s, 1H, CH-(OR)₂], 5.35 (s, 1H, OH), 7.21 (d, 2H, J = 8.0 Hz Aromatenprotonen), 7.31 (d, 2H, J = 8.4 Hz, Aromatenprotonen)

Anschließende Hydrolyse zum 4-(17β-Ethoxy-3-oxoestra-4,9-dien-11β-yl)benzaldehyd
Schmelzpunkt.: 149 bis 152 °C (tert.-Butylmethylether)
α_{D} = + 216° (CHCl₃);
¹H-NMR-Spektrum in CDCl₃ [δ, ppm]: 0.42 (s, 3H, H-18), 1.16 (t, 3H, Ethyl-CH₃), 3.31 (t, 1 H, J=8.4 Hz,17α-H), 3.48 (q, 2H, CH₂CH₃), 4.40 (d, J=7.6 Hz, 1 H, H-11 α), 5.78 (s, 1 H, H-4), 7.37 (d, 2H, J = 8.0 Hz, Aromatenprotonen), 7.80 (d, 2H, J = 8.4 Hz, Aromatenprotonen), 8.96 (s, 1 H, CH=O).

### Beispiel 16

### 4-(17β-Benzoyloxy-3-oxoestra-4,9-dien-11β-yl)benzaldehyd-1(E)-oxim

Analog Beispiel 3 aus 4-(17β-Hydroxy-3-oxoestra-4,9-dien-11β-yl)benzaldehyd und Benzoylchlorid in Pyridin und anschließende Oximierung mit Hydroxylaminhydrochlorid in Pyridin nach Beispiel 1.
Schmelzpunkt: 132 bis 133 °C ( Aceton )
α_{D} = + 219° (CHCl₃);
¹H-NMR-Spelctrum in CDCl₃ [δ, ppm]: 0.61 (s, 3H, H-18), 4.38 (d, J=7.6 Hz, 1H, H-11α), 4.89 (t, 1H, 17α-H), 5.79 (s, 1H, H-4), 7.19 (d, 2H, J = 8.4 Hz, Aromatenprotonen), 7.40-7.55 (m, 5H, Aromat) 7.99 (d, 2H, J = 8.0 Hz, Aromatenprotonen), 8.09 (s, 1 H, CH=N-).

### Beispiel 17

### 4-(17β-Benzyloxy-3-oxoestra-4,9-dien-11β-yl)benzaldehyd-1(E)-oxim

Analog Beispiel 1 aus 4-(17β-Benzyloxy-3-oxoestra-4,9-dien-11β-yl)benzaldehyd und Hydroxylaminhydrochloird in Pyridin.
Schmelzpunkt: 103 bis 109 °C (Aceton)
¹H-NMR-Spektrum in CDCl₃ [δ, ppm]: 0.51 (s, 3H, H-18), 3.42 (t, J=8.0 Hz, H-17α), 4.33 (d, J=7.6 Hz, 1H, H-11α), 4.49 (q, 2H, PhCH₂), 5.76 (s, 1H, H-4), 7.19 (d, 2H, J = 8.0 Hz, Aromatenprotonen), 7.25-7.34 (m, 5H, Aromat) 7.48 (d, 2H, J = 8.4 Hz, Aromatenprotonen), 8.09 (s, 1 H, CH=N-).

### Herstellung des Ausgangsmaterials

790 mg [4-(3,3-Dimethoxy-5α17β-dihydroxy-estr-9-en-11β-yl)benzaldehydneopentylketal] werden in 15 ml Toluen mit 1 ml Benzylbromid in Gegenwart von 675 mg Kalium tert.-butanolat nach Beispiel 5 umgesetzt. Das Rohprodukt wird in 8 ml Aceton mit 100 mg p-Toluolsulfonsäure innerhalb von 3 Stunden zum 4-(17β-Benzyloxy-3-oxoestra-4,9-dien-11β-yl)benzaldehyd hydrolysiert.
Schmelzpunkt: 83 bis 87 °C (Aceton)
¹H-NMR-Spektrum in CDCl₃ [δ, ppm]: 0.48 (s, 3H, H-18), 3.42 (t, 1H, H-17α), 4.39 (d, J=7.6 Hz, 1H, H-11α), 4.52 (q, 2H, J= 12,0 Hz, J= 35,6 Hz, CH₂Ph), 5.77 (s, 1 H, H-4), 7.29-7.35 (m, 5H, Aromat), 7.36 (d, 2H, J = 8.4 Hz, Aromatenprotonen), 7.80 (d, 2H, J = 8.0 Hz, Aromatenprotonen), 9.96 (s, 1 H, CH=O).

### Beispiel 18

### 4-(17β-Methoxy-3-oxoestra-4,9-dien-11β-yl)benzaldehyd-1(E)-[(N-ethyl)-carbonyl]oxim

Analog Beispiel 1 aus 4-(17β-Methoxy-3-oxoestra-4,9-dien-11β-yl]benzaldehyd-1-(E)-oxim mit Ethylisocyanat in Toluen.
Schmelzpunkt: 175 bis 176 °C
α_{D} = + 291° (CHCl₃)
¹H-NMR-Spektrum in CDCl₃ [δ, ppm]: 0.42 (s, 3H, H-18), 1.23 (t, 3H, Ethyl), 3.25 (t, 1 H, H-17α), 3.34 (s, 3H, OCH₃), 3.37 (q, 2H, CH₂CH₃), 4.38 (d, J=7.6 Hz, 1 H, H-11α), 5.78 (s, 1 H, H-4), 6.21 (t, 1 H, NH), 7.26 (d, 2H, J = 8.4 Hz, Aromatenprotonen), 7.58 (s, 2H, J = 8.0 Hz, Aromatenprotonen), 8.28 (s, 1 H, CH=N-).

### Beispiel 19

### 4-(4'-Brom-17β-hydroxy-3-oxoestra-4,9-dien-11β-yl)benzaldehyd-1(E)-oxim

Analog Beispiel 1 aus 4-(4'-Brom-17β-hydroxy-3-oxoestra-4,9-dien-11β-yl)benzaldehyd und Hydroxylaminhydrochlorid in Pyridin.
Schmelzpunkt: ab 273 °C (Zersetzung, Ether)
α_{D} = + 209 ° (CHCl₃)
1H-NMR-Spektrum in CDCl₃ [δ, ppm] 0.44 (s, 3H, H-18), 3.68 (t, 1 H, H-17α), 4.36 (d, J = 6.8 Hz, 1 H, H-11α), 7.17 und 7.47 (2d, 4H, J= 8.0 Hz, A,A', B,B'-System der Aromatenprotonen), 8.09 (s, 1 H, CH=N-), 8.35 (s, 1H, NOH).

### Herstellung der Ausgangsverbindung

4-(17β-Hydroxy-3-oxoestra-4,9-dien-11β-yl)benzaldehyd werden nach Beispiel 5 in THF mit NBS zum 4-(4'-Brom-17β-hydroxy-3-oxoestra-4,9-dien-11β-yl)benzaldehyd umgesetzt und durch Chromatographie gereinigt. Das gereinigte Produkt wird direkt in die Oximierung eingesetzt.
¹H-NMR-Spektrum in CDCl₃ [δ, ppm]: 0.52 (s, 3H, H-18), 1.27 (s, 3H, 17α-CH₃), 3,21 (2t, 1 H, OH), 4.41 (d, J = 7.2 Hz, 1 H, H-11α), 7.34 und 7.79 (2d, 4 H, J= 8.0 Hz, A,A', B,B'-System der Aromatenprotonen), 9.96 (s, 1 H, CH=O).

### Beispiel 20

### 4-[17β-Hydroxy-17α-hydroxymethyl-3-oxoestra-4,9-dien-11β-yl]benzaldehyd-1-(E)-[O-(ethylamino)-carbonyl]oxim

1,5 g 4-[17β-Hydroxy-17α-(tetrahydrohydropyranoyloxy)methyl-3-oxoestra-4,9-dien-11β-yl]benzaldehyd-1-(E)-[O-(ethylamino)-carbonyl]oxim werden in 30 ml Eisessig gelöst und 24h bei 50°C Tage gerührt. Anschließend tropft man in wässrige NaHCO₃-Lösung ein, extrahiert mit Essigester, wäscht die organische Phase mit Wasser und trocknet über Na₂SO₄. Das Lösungsmittel wird unter Vakuum verdampft und der kristalline Rückstand (1,33 g) durch Flashchromatographie an Kieselgel mit einem Toluen/Aceton Gemisch gereinigt.
Schmelzpunkt.: 164 bis 166°C ( Aceton)
α_{D} = + 258° (CHCl₃)
¹H-NMR-Spektrum in CDCl₃ + D₂O [δ, ppm]: 0.55 (s, 3H, H-18), 1.23 (t, 3H, CH₂CH₃), 1.62 (s, 3H, CH₃), 3.37 (m, 2H, CH₂), 3,35 und 3,79 (2d, 2H, J=10,8 Hz, CH₂OH), 4.42 (d, J=7.2 Hz, 1 H, H-11α), 5.78 (s, 1 H, H-4), 7.26 (d, 2H, J = 8.0 Hz, Aromatenprotonen), 7.58 (d, 2H, J = 8.0 Hz, Aromatenprotonen), 8.28 (s, 1H, CH=NOR).

### Herstellung der Ausgangsverbindung

### Stufe 1

Analog Beispiel 9 wird 4-(3,3-Dimethoxy-5α-hydroxy-17-oxoestr-9-en-11β-yl)-benzaldehyd-ethylenacetal in das 4-(3,3-Dimethoxy-5α-hydroxy-17-(S)-spiro-epoxy-estr-9-en-11β-yl)benzaldehyd-ethylenacetal überführt.

19,4 g 4-(3,3-Dimethoxy-5α-hydroxy-17-(S)-spiroepoxy-estr-9-en-11β-yl)benzaldehyd-ethylenacetal werden in 250ml N-Methyl-2-pyrrolidon gelöst. Man tropft 145 ml 2N wässrige NaOH zu, erhitzt 2 h auf 100°C, kühlt ab und tropft in 250 ml wässrige 10 % ige NH₄Cl-Lösung ein. Nach Extraktion mit Essigester wird die organische Phase neutral gewaschen, getrocknet und unter Vakuum verdampft. Man erhält 19,5 g (Ausbeute 65 %) 4-(3,3-Dimethoxy-5α,17β-dihydroxy-17α-hydroxymethyl-estr-9-en-11β-yl)benzaldehyd-ethylenacetal als Rohprodukt. ¹H-NMR-Spektrum in CDCl₃ + D₂O [δ, ppm]: 0.47 (s, 3H, H-18), 3,21 und 3.22 (2s, je 3H, OCH₃), 3,40 und 3,74 (2d, 2H, J=10,8 Hz, CH₂OH), 4.07 (m, 4H, Ethylenacetal), 4.42 (d, J=7.2 Hz, 1 H, H-11α), 5.76 (s, 1 H, Benzaldehydacetal), 7.23 (d, 2H, J = 8.0 Hz, Aromatenprotonen), 7.37 (d, 2H, J = 8.0 Hz, Aromatenprotonen).

Dieses Produkt wird in 120 ml Tetrahydrofuran gelöst, mit 12 ml Wasser und 5,3 g p-Toluolsulfonsäure versetzt. 4 h bei Raumtemperatur gerührt. Anschließend neutralisiert man die Lösung mit wässriger NaHCO₃-Lösung und isoliert mit Essigester nach dem üblichen Aufarbeitungsvertahren 8,5 g 4-[17β-Hydroxy-17α-(hydroxymethyl)-3-oxoestra-4,9-dien-11β-yl]benzaldehyd als einen hellgelben Schaum, der durch Flashchromatographie gereinigt wird (Ausbeute 65 %).
Schmelzpunkt.: 116 bis 123°C ( Aceton)
α_{D} = + 185° (CHCl₃)
¹H-NMR-Spektrum in CDCl₃ [δ, ppm]: 0.60 (s, 3H, H-18), 3,43 und 3,79 (2d, 2H, J=10,8 Hz, CH₂OH), 4.32 (d, J=7.4 Hz, 1 H, H-11α), 5.74 (s, 1 H, H-4), 6.67 (d, 2H, J = 8.0 Hz, Aromatenprotonen), 7.00 (d, 2H, J = 8.0 Hz, Aromatenprotonen) 9.98 (CHO).

### Stufe2

5,4 g 4-[17β-Hydroxy-17α-(hydroxymethyl)-3-oxoestra-4,9-dien-11β-yl]benzaldehyd werden in 50 ml Methylenchlorid mit 16,9 ml 3,4-Dihydro-2H-pyran und 335 mg Pyridinium-4-toluoisuifonat innerhalb 1 h bei Raumtemperatur umgesetzt. Anschließend rührt man die Lösung in 100 ml gesättigte wässrige NaHCO₃-Lösung ein, extrahiert mit Methylenchlorid, wäscht die organische Lösung neutral, trocknet über Na₂SO₄, filtriert ab und engt das Lösungsmittel unter Vakuum ein. Man er hält 7,3 g eines Gemisches aus 4-[17β-Hydroxy-17α-(tetrahydroxypyranyloxy)methyl-3-oxoestra-4,9-dien-11β-yl]benzaldehyd und 4-[17β-Tetrahydropyranyloxy,17α-(tetrahydroxypyranyloxy)methyl-3-oxoestra-4,9-dien-11 β-yl]benzaldehyd. Nach Flashchromatographie an Kieselgel mit einem Toluen/Aceton-Gradienten werden 4,7 g 4-[17β-Hydroxy-17α-(tetrahydroxypyranyloxy)methyl-3-oxoestra-4,9-dien-11β-yl]benzaldehyd isoliert. Reinheit LC/MS: 491 (M⁺ + 1) 99 % Fläche (Isomerengemisch, THP-monoether 87% und 12 %)
¹H-NMR-Spektrum in CDCl₃ [δ, ppm]: 0.53 (s, 3H, H-18), 4.44 (d, J=6.4 Hz, 1 H, H-11α), 4.56 und 4.60 (2t, 2H, CH₂OH), 5.79 (s, 1 H, H-4), 7.36 (d, 2H, J = 8.0 Hz, Aromatenprotonen), 7.79 (d, 2H, J = 8.0 Hz, Aromatenprotonen) 9.95 (CHO).

### Stufe 3

Analog Beispiel 1 werden aus 4,75 g 4-[17β-Hydroxy-17α-(tetrahydroxypyranyloxy)-methyl-3-oxoestra-4,9-dien-11β-yl]benzaldehyd und 603 mg Hydroxylaminhydro-chlorid in 45 ml Pyridin 4,8 g 4-[17β-Hydroxy-17α-(tetrahydroxypyranyloxy)methyl-3-oxoestra-4,9-dien-11β-yl]benzaldehydoxim erhalten, die durch Flashchromatographie an Kieselgel mit einem Toluen/Aceton-Gradienten gereinigt werden.
Reinheit LC/MS: 491 (M⁺ + 1) 99 % Fläche (Isomerengemisch, THP-monoether - 93 % und 6 %)
¹H-NMR-Spektrum in CDCl₃ [δ, ppm]: 0.88 (s, 3H, H-18), 4.44 (d, J=6.4 Hz, 1 H, H-11α), 4.55 und 4.60 (2t, 2H, CH₂OH), 5.77 (s, 1H, H-4), 7.19 (d, 2H, J = 8.4 Hz, Aromatenprotonen), 7.46 (d, 2H, J = 8.4 Hz, Aromatenprotonen) 8.09 (CH=NOH).

### Stufe 4

Analog Beispiel 4 werden aus 1,32 g 4-[17β-Hydroxy-17α-(tetrahydroxypyranyloxy)methyl-3-oxoestra-4,9-dien-11β-yl]benzaldehydoxim mit Ethylisocyanat in Toluol 1,54 g 4-[17β-Hydroxy-17α-(tetrahydrohydropyranoyloxy)methyl-3-oxo-estra-4,9-dien-11β-yl]benzaldehyd-1-(E)-[O-(ethylamino)-carbonyl]oxim als Rohprodukt erhalten, die ohne weitere Reinigung in die Endstufe eingesetzt werden. ¹H-NMR-Spektrum in CDCl₃ [δ, ppm]: 0.55 (s, 3H, H-18), 1.23 (t. 3H. CH₂CH₃), 3,4 (m, 2 H, CH₂CH₃), 4.44 (m, 1H, H-11α), 4.6 und 4.90 (2m, 2H, CH₂OR), 5.77 (s, 1 H, H-4), 6.22 (s, 1 H, NH), 7.26 (d, 2H, J = 8.4 Hz, Aromatenprotonen), 7.57 (d, 2H, J = 8.4 Hz, Aromatenprotonen) 8.29 (CH=NOR).

## Patentansprüche

1. Verwendung von Verbindungen der allgemeinen Formel I 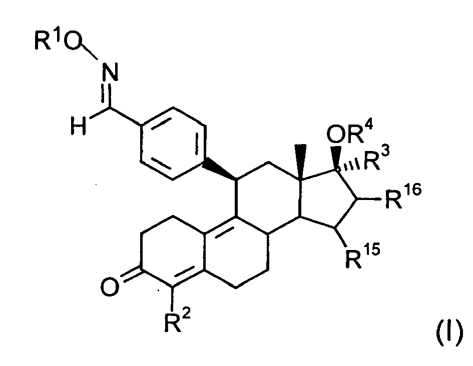 worin die Reste R¹, R², R³, R⁴ und R⁵ sowie R¹⁵ und R¹⁶ folgende Bedeutung besitzen:
R¹ ein Wasserstoffatom, ein Alkanoylrest mit 1 bis 10 Kohlenstoffatomen oder ein gegebenenfalls substituierter Benzoylrest mit 6-10 Kohlenstoffatomen oder ein Rest CONHR⁵, wobei
R⁵ ein Wasserstoffatom, ein Alkyl- oder Acylrest mit jeweils 1-10 Kohlenstoffatomen oder ein Alkylaryl- oder Aralkylrest mit jeweils 6-10 Kohlenstoffatomen ist,
R² ein Wasserstoffatom, ein Halogenatom oder eine CF₃-Gruppe,
R³ ein Wasserstoffatom oder eine Gruppe CH₂X, in der
X für ein Wasserstoffatom, eine Hydroxygruppe, ein Halogenatom, einen Alkylrest mit 1 oder 2 Kohlenstoffatomen, für einen Rest (CH₂)ₙCH₂Y mit n = 0 oder 1 und Y für ein Halogenatom steht,
wobei, wenn
R² ein Halogenatom ist, R³ zusätzlich eine Gruppe CₙFₘHₒ bedeuten kann, wobei n = 1, 2, 3, 4 oder 5, m > 1 und
m + o = 2n + 1 ist,
R⁴ ein Wasserstoffatom, Alkyl- oder Alkanoylrest aus jeweils 1-10 Kohlenstoffatomen oder ein Benzoylrest mit 6-10 Kohlenstoffatomen oder ein Rest -CONHR⁵, wobei R⁵ die oben angegebene Bedeutung besitzt,
R¹⁵ und R¹⁶ Wasserstoffatome oder gemeinsam eine Doppelbindung darstellen,
und ihrer pharmazeutisch annehmbaren Salze,
zur Herstellung von Arzneimitteln zur Behandlung von dysfunktionellen Blutungen.

2. Verwendung von Verbindungen der allgemeinen Formel I 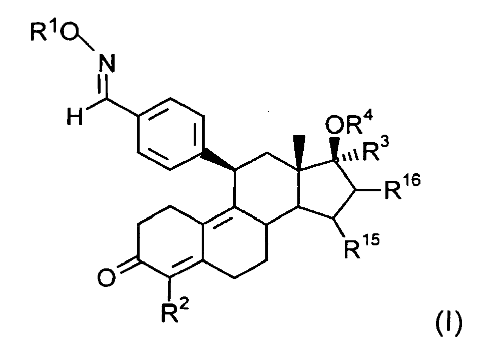 worin die Reste R¹, R², R³, R⁴ und R⁵ sowie R¹⁵ und R¹⁶ folgende Bedeutung besitzen:
R¹ ein Wasserstoffatom, ein Alkanoylrest mit 1 bis 10 Kohlenstoffatomen oder ein gegebenenfalls substituierter Benzoylrest mit 6-10 Kohlenstoffatomen oder ein Rest CONHR⁵, wobei
R⁵ ein Wasserstoffatom, ein Alkyl- oder Acylrest mit jeweils 1-10 Kohlenstoffatomen oder ein Alkylaryl- oder Aralkylrest mit jeweils 6-10 Kohlenstoffatomen ist,
R² ein Wasserstoffatom, ein Halogenatom oder eine CF₃-Gruppe,
R³ ein Wasserstoffatom oder eine Gruppe CH₂X, in der X für ein Wasserstoffatom, eine Hydroxygruppe, ein Halogenatom, einen Alkylrest mit 1 oder 2 Kohlenstoffatomen, für einen Rest (CH₂)ₙCH₂Y mit n = 0 oder 1 und Y für ein Halogenatom steht, wobei, wenn
R² ein Halogenatom ist, R³ zusätzlich eine Gruppe CₙFₘHₒ bedeuten kann, wobei n = 1, 2, 3, 4 oder 5, m > 1 und
m + o = 2n + 1 ist,
R⁴ ein Wasserstoffatom, Alkyl- oder Alkanoylrest aus jeweils 1-10 Kohlenstoffatomen oder ein Benzoylrest mit 6-10 Kohlenstoffatomen oder ein Rest -CONHR⁵, wobei R⁵ die oben angegebene Bedeutung besitzt,
R¹⁵ und R¹⁶ Wasserstoffatome oder gemeinsam eine Doppelbindung darstellen,
und ihrer pharmazeutisch annehmbaren Salze,
zur Herstellung von Arzneimitteln zur Behandlung von Dysmenorrhoe.

3. Verwendung von Verbindungen der allgemeinen Formel I 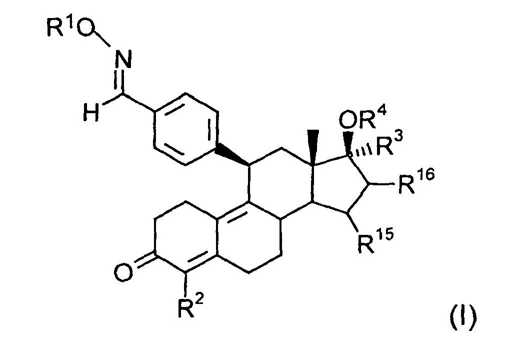 worin die Reste R¹, R², R³, R⁴ und R⁵ sowie R¹⁵ und R¹⁶ folgende Bedeutung besitzen:
R¹ ein Wasserstoffatom, ein Alkanoylrest mit 1 bis 10 Kohlenstoffatomen oder ein gegebenenfalls substituierter Benzoylrest mit 6-10 Kohlenstoffatomen oder ein Rest CONHR⁵, wobei
R⁵ ein Wasserstoffatom, ein Alkyl- oder Acylrest mit jeweils 1-10 Kohlenstoffatomen oder ein Alkylaryl- oder Aralkylrest mit jeweils 6-10 Kohlenstoffatomen ist,
R² ein Wasserstoffatom, ein Halogenatom oder eine CF₃-Gruppe,
R³ ein Wasserstoffatom oder eine Gruppe CH₂X, in der X für ein Wasserstoffatom, eine Hydroxygruppe, ein Halogenatom, einen Alkylrest mit 1 oder 2 Kohlenstoffatomen, für einen Rest (CH₂)ₙCH₂Y mit n = 0 oder 1 und Y für ein Halogenatom steht, wobei, wenn
R² ein Halogenatom ist, R³ zusätzlich eine Gruppe CₙFₘHₒ bedeuten kann, wobei n = 1, 2, 3, 4 oder 5, m > 1 und
m + o = 2n + 1 ist,
R⁴ ein Wasserstoffatom, Alkyl- oder Alkanoylrest aus jeweils 1-10 Kohlenstoffatomen oder ein Benzoylrest mit 6-10 Kohlenstoffatomen oder ein Rest -CONHR⁵, wobei R⁵ die oben angegebene Bedeutung besitzt,
R¹⁵ und R¹⁶ Wasserstoffatome oder gemeinsam eine Doppelbindung darstellen,
und ihrer pharmazeutisch annehmbaren Salze,
zur Herstellung von Arzneimitteln zur Induktion einer Amenorrhoe.

4. Verwendung von Verbindungen der allgemeinen Formel I 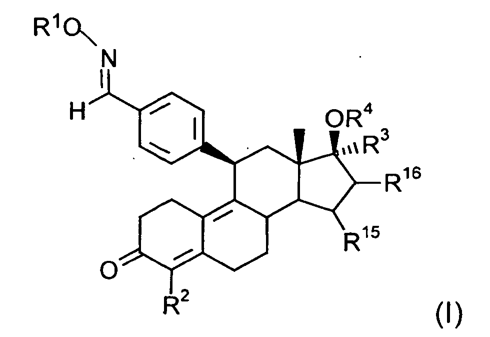 worin die Reste R¹, R², R³, R⁴ und R⁵ sowie R¹⁵ und R¹⁶ folgende Bedeutung besitzen:
R¹ ein Wasserstoffatom, ein Alkanoylrest mit 1 bis 10 Kohlenstoffatomen oder ein gegebenenfalls substituierter Benzoylrest mit 6-10 Kohlenstoffatomen oder ein Rest CONHR⁵, wobei
R⁵ ein Wasserstoffatom, ein Alkyl- oder Acylrest mit jeweils 1-10 Kohlenstoffatomen oder ein Alkylaryl- oder Aralkylrest mit jeweils 6-10 Kohlenstoffatomen ist,
R² ein Wasserstoffatom, ein Halogenatom oder eine CF₃-Gruppe,
R³ ein Wasserstoffatom oder eine Gruppe CH₂X, in der
X für ein Wasserstoffatom, eine Hydroxygruppe, ein Halogenatom, einen Alkylrest mit 1 oder 2 Kohlenstoffatomen, für einen Rest (CH₂)ₙCH₂Y mit n = 0 oder 1 und Y für ein Halogenatom steht, wobei, wenn
R² ein Halogenatom ist, R³ zusätzlich eine Gruppe CₙFₘHₒ bedeuten kann, wobei n = 1, 2, 3, 4 oder 5, m > 1 und
m + o = 2n + 1 ist,
R⁴ ein Wasserstoffatom, Alkyl- oder Alkanoylrest aus jeweils 1-10 Kohlenstoffatomen oder ein Benzoylrest mit 6-10 Kohlenstoffatomen oder ein Rest -CONHR⁵, wobei R⁵ die oben angegebene Bedeutung besitzt,
R¹⁵ und R¹⁶ Wasserstoffatome oder gemeinsam eine Doppelbindung darstellen,
und ihrer pharmazeutisch annehmbaren Salze,
zur Herstellung von Arzneimitteln zur Behandlung von hormonellen Störungen bei postmenopausalen Frauen.

5. Verwendung gemäß einem der Ansprüche 1 bis 2 in Kombination mit mindestens einem niedrig dosierten natürlichen oder synthetischen Estrogen oder deren Prodrugs.

6. Verwendung gemäß Anspruch 5 **dadurch gekennzeichnet, dass** das Estrogen als 3-Sulfamat eingesetzt wird.

7. Verwendung gemäß Anspruch 6 **dadurch gekennzeichnet, dass** das Estrogen-3-sulfamat 17β-Hydroxy-estra-1,3,5(10)-trien-3yl-sulfamat ist.

8. Verbindungen der allgemeinen Formel I 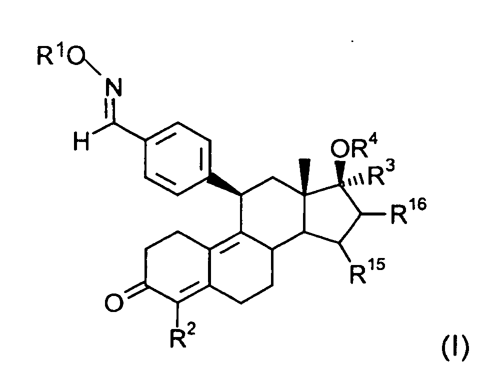 worin die Reste R¹, R², R³, R⁴ und R⁵ sowie R¹⁵ und R¹⁶ folgende Bedeutung besitzen:
R¹ ein Wasserstoffatom, ein Alkanoylrest mit 1 bis 10 Kohlenstoffatomen oder ein gegebenenfalls substituierter Benzoylrest mit 6-10 Kohlenstoffatomen oder ein Rest CONHR⁵, wobei
R⁵ ein Wasserstoffatom, ein Alkyl- oder Acylrest mit jeweils 1-10 Kohlenstoffatomen oder ein Alkylaryl- oder Aralkylrest mit jeweils 6-10 Kohlenstoffatomen ist,
R² ein Halogenatom oder eine CF₃-Gruppe,
R³ ein Wasserstoffatom oder eine Gruppe CH₂X, in der
X für ein Wasserstoffatom, eine Hydroxygruppe, ein Halogenatom, einen Alkylrest mit 1 oder 2 Kohlenstoffatomen, für einen Rest (CH₂)ₙCH₂Y mit n = 0 oder 1 und Y für ein Halogenatom steht, wobei, wenn
R² ein Halogenatom ist, R³ zusätzlich eine Gruppe CₙFₘHₒ bedeuten kann, wobei n = 1, 2, 3, 4 oder 5, m > 1 und
m + o = 2n + 1 ist,
R⁴ ein Wasserstoffatom, Alkyl- oder Alkanoylrest aus jeweils 1-10 Kohlenstoffatomen oder ein Benzoylrest mit 6-10 Kohlenstoffatomen oder ein Rest -CONHR⁵, wobei R⁵ die oben angegebene Bedeutung besitzt,
R¹⁵ und R¹⁶ Wasserstoffatome oder gemeinsam eine Doppelbindung darstellen,
und ihre pharmazeutisch annehmbaren Salze.

9. Verbindungen der allgemeinen Formel I nach Anspruch 8, worin R² ein Chlor- oder Bromatom ist.

10. Verbindungen der allgemeinen Formel I nach Anspruch 8, worin R³ ein Wasserstoffatom oder eine Gruppe CH₂X,
in der X ein Wasserstoffatom, eine Hydroxygruppe, ein Halogenatom, ein Alkylrest mit 1 oder 2 Kohlenstoffatomen, ein Rest (CH₂)ₙCH₂Y mit n = 0 oder 1 und Y ein Halogenatom sein kann,
und X und/ oder Y Fluor, Chlor oder Brom sein können.

11. Verbindungen der allgemeinen Formel I nach Anspruch 8, **dadurch gekennzeichnet, dass** R⁴ ein Wasserstoffatom oder ein Alkylrest mit 1 bis 4 Kohlenstoffatomen ist.

12. Verbindungen der allgemeinen Formel I nach Anspruch 8, worin R¹ ein Wasserstoffatom bedeutet, R² für ein Chlor- oder ein Bromatom steht und R³ ein Wasserstoffatom, eine Methylgruppe, eine CH₂X-Gruppe sein kann, wobei X für ein Fluor-, Chlor- oder Bromatom oder eine Hydroxygruppe steht.

13. Verbindungen der allgemeinen Formel I nach Anspruch 8, nämlich:
4-[4'-Brom-17β-hydroxy-3-oxoestra-4,9-dien-11β-yl]benzäldehyd-1-(E)-oxim,
4-[4'-Brom-17β-hydroxy-17α-methyl-3-oxoestra-4,9-dien-11β-yl]benzaldehyd-1-(E)-oxim,
4-[4'-Brom-17β-hydroxy-17α-trifluormethyl-3-oxoestra-4,9-dien-11β-yl]-benzaldehyd-1-(E)-oxim,
4-[17β-Acetoxy-4'-brom-3-oxoestra-4,9-dien-11β-yl]benzaldehyd-1-(E)-oxim,
4-[17β-Acetoxy-4'-brom-3-oxoestra-4,9-dien-11β-yl]benzaldehyd-1-(E)-O-acetyloxim,
4-[4'-Brom-17β-methoxy-3-oxoestra-4,9-dien-11β-yl]benzaldehyd-1-(E)-oxim,
4-[4'-Chlor-17β-hydroxy-17α-trifluormethyl-3-oxoestra-4,9-dien-11β-yl]benzaldehyd-1-(E)-oxim,
4-[4'-Brom-17α-fluormethyl-17β-hydroxy-3-oxoestra-4,9-dien-11β-yl]-benzaldehyd-1-(E)-oxim,
4-[4'-Brom-17α-chlormethyl-17β-hydroxy-3-oxoestra-4,9-dien-11β-yl]-benzaldehyd-1-(E)-oxim,
4-[4'-Brom-17α-brommethyl-17β-hydroxy-3-oxoestra-4,9-dien-11β-yl]-benzaldehyd-1-(E)-oxim,
4-[4'-Chlor-17β-methoxy-3-oxoestra-4,9-dien-11β-yl]benzaldehyd-1-(E)-oxim,
4-[4'-Chlor-17α-chlormethyl-17β-hydroxy-3-oxoestra-4,9-dien-11β-yl]-benzaldehyd-1-(E)-oxim,
4-[17β-Methoxy-4'-trifluormethyl-3-oxoestra-4,9-dien-11β-yl]benzaldehyd-1-(E)-oxim,
4-[4'-Chlor-17β-hydroxy-17α-methyl-3-oxoestra-4,9-dien-11β-yl]benzaldehyd-1 (E)-oxim,

14. Pharmazeutische Präparate enthaltend mindestens eine Verbindung der allgemeinen Formel I gemäß Anspruch 8 sowie einen pharmazeutisch verträglichen Träger.

15. Verwendung der Verbindungen der allgemeinen Formel I nach einem der Ansprüche 8 bis 13 zur Herstellung von Arzneimitteln zur Behandlung der Endometriose sowie des Uterus myomatoses.

16. Verwendung von Verbindungen der allgemeinen Formel I nach einem der Ansprüche 8 bis 13 zur Herstellung von Arzneimitteln für die weibliche Fertilitätskontrolle.

17. Verwendung von Verbindungen der allgemeinen Formel I 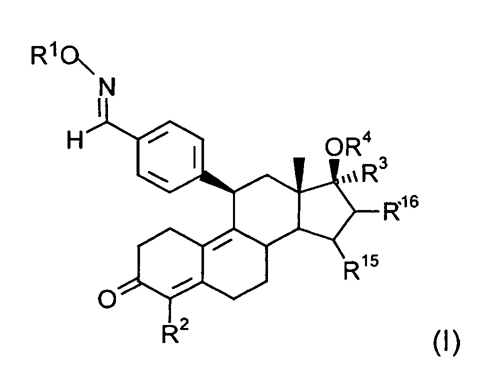 worin die Reste R¹, R², R³, R⁴ und R⁵ sowie R¹⁵ und R¹⁶ folgende Bedeutung besitzen:
R¹ ein Wasserstoffatom, ein Alkanoylrest mit 1 bis 10 Kohlenstoffatomen oder ein gegebenenfalls substituierter Benzoylrest mit 6-10 Kohlenstoffatomen oder ein Rest CONHR⁵, wobei
R⁵ ein Wasserstoffatom, ein Alkyl- oder Acylrest mit jeweils 1-10 Kohlenstoffatomen oder ein Alkylaryl- oder Aralkylrest mit jeweils 6-10 Kohlenstoffatomen ist,
R² ein Wasserstoffatom,
R³ ein Wasserstoffatom oder eine Gruppe CH₂X, in der
X für ein Wasserstoffatom, eine Hydroxygruppe, ein Halogenatom, einen Alkylrest mit 1 oder 2 Kohlenstoffatomen, für einen Rest (CH₂)ₙCH₂Y mit n = 0 oder 1 und Y für ein Halogenatom steht, wobei, wenn
R² ein Halogenatom ist, R³ zusätzlich eine Gruppe CₙFₘHₒ bedeuten kann, wobei n = 1, 2, 3, 4 oder 5, m > 1 und
m + o = 2n + 1 ist,
R⁴ ein Wasserstoffatom, Alkyl- oder Alkanoylrest aus jeweils 1-10 Kohlenstoffatomen oder ein Benzoylrest mit 6-10 Kohlenstoffatomen oder ein Rest -CONHR⁵, wobei R⁵ die oben angegebene Bedeutung besitzt,
R¹⁵ und R¹⁶ Wasserstoffatome oder gemeinsam eine Doppelbindung darstellen,
und ihrer pharmazeutisch annehmbaren Salze,
zur Herstellung von Arzneimitteln für die weibliche Fertilitätskontrolle.

18. Verwendung gemäß Anspruch 16 oder 17 in Kombination mit mindestens einem niedrig dosierten natürlichen oder synthetischen Estrogen oder deren Prodrugs.

19. Verwendung gemäß Anspruch 18 **dadurch gekennzeichnet, dass** das Estrogen als 3-Sulfamat eingesetzt wird.

20. Verwendung gemäß Anspruch 19 **dadurch gekennzeichnet, dass** das Estrogen-3-sulfamat 17β-Hydroxy-estra-1,3,5(10)-trien-3yl-sulfamat ist.

## Claims

1. Use of compounds of general formula I 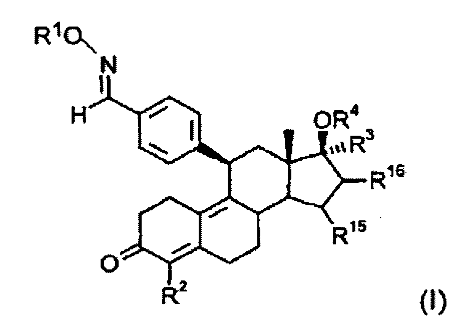 wherein the residues R¹, R², R³, R⁴, R⁵, as well as R¹⁵ and R¹⁶, have the following meanings:
R¹ represents a hydrogen atom, an alkanoyl residue with 1 to 10 carbon atoms or an optionally substituted benzoyl residue with 6-10 carbon atoms or a CONHR⁵ residue, wherein
R⁵ is a hydrogen atom, an alkyl or acyl residue with 1-10 carbon atoms, or an alkylaryl or aralkyl residue with 6-10 carbon atoms,
R² represents a hydrogen atom, a halogen atom or a CF₃ group,
R³ represents a hydrogen atom or a CH₂X group wherein
X represents a hydrogen atom, a hydroxy group, a halogen atom, an alkyl residue with 1 or 2 carbon atoms, a (CH₂)ₙCH₂Y residue wherein n = 0 or 1 and Y represents a halogen atom, and
if R² is a halogen atom, R³ additionally can be a CₙFₘHₒ group wherein n = 1, 2, 3, 4 or 5, m > 1, and m + o = 2n + 1,
R⁴ represents a hydrogen atom, an alkyl or alkanoyl residue with 1-10 carbon atoms or a benzoyl residue with 6-10 carbon atoms or a -CONHR⁵ residue wherein R⁵ has the meaning specified above,
R¹⁵ and R¹⁶ represent hydrogen atoms or, taken together, a double bond,
and pharmaceutically acceptable salts thereof,
in the production of drugs for the treatment of dysfunctional bleeding.

2. Use of compounds of general formula I 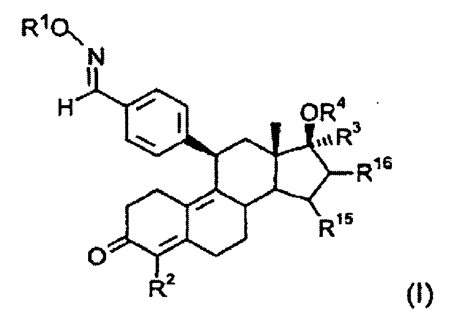 wherein the residues R¹, R², R³, R⁴, R⁵, as well as R¹⁵ and R¹⁶, have the following meanings:
R¹ represents a hydrogen atom, an alkanoyl residue with 1 to 10 carbon atoms or an optionally substituted benzoyl residue with 6-10 carbon atoms or a CONHR⁵ residue, wherein
R⁵ is a hydrogen atom, an alkyl or acyl residue with 1-10 carbon atoms, or an alkylaryl or aralkyl residue with 6-10 carbon atoms,
R² represents a hydrogen atom, a halogen atom or a CF₃ group,
R³ represents a hydrogen atom or a CH₂X group wherein
X represents a hydrogen atom, a hydroxy group, a halogen atom, an alkyl residue with 1 or 2 carbon atoms, a (CH₂)ₙCH₂Y residue wherein n = 0 or 1 and Y represents a halogen atom, and
if R² is a halogen atom, R³ additionally can be a CₙFₘHₒ group wherein n = 1, 2, 3, 4 or 5, m > 1, and m + o = 2n + 1,
R⁴ represents a hydrogen atom, an alkyl or alkanoyl residue with 1-10 carbon atoms or a benzoyl residue with 6-10 carbon atoms or a -CONHR⁵ residue wherein R⁵ has the meaning specified above,
R¹⁵ and R¹⁶ represent hydrogen atoms or, taken together, a double bond,
and pharmaceutically acceptable salts thereof,
in the production of drugs for the treatment of dysmenorrhea.

3. Use of compounds of general formula I 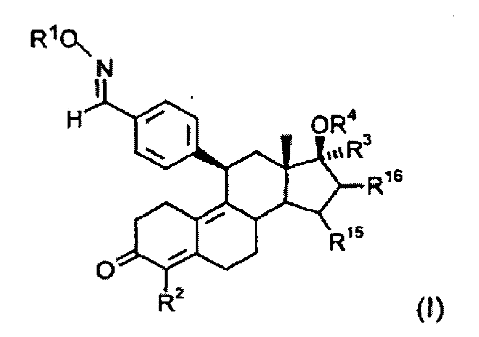 wherein the residues R¹, R², R³, R⁴, R⁵, as well as R¹⁵ and R¹⁶, have the following meanings:
R¹ represents a hydrogen atom, an alkanoyl residue with 1 to 10 carbon atoms or an optionally substituted benzoyl residue with 6-10 carbon atoms or a CONHR⁵ residue, wherein
R⁵ is a hydrogen atom, an alkyl or acyl residue with 1-10 carbon atoms, or an alkylaryl or aralkyl residue with 6-10 carbon atoms,
R² represents a hydrogen atom, a halogen atom or a CF₃ group,
R³ represents a hydrogen atom or a CH₂X group wherein
X represents a hydrogen atom, a hydroxy group, a halogen atom, an alkyl residue with 1 or 2 carbon atoms, a (CH₂)ₙCH₂Y residue wherein n = 0 or 1 and Y represents a halogen atom, and
if R² is a halogen atom, R³ additionally can be a CₙFₘHₒ group wherein n = 1, 2, 3, 4 or 5, m > 1, and m + o = 2n + 1,
R⁴ represents a hydrogen atom, an alkyl or alkanoyl residue with 1-10 carbon atoms or a benzoyl residue with 6-10 carbon atoms or a -CONHR⁵ residue wherein R⁵ has the meaning specified above,
R¹⁵ and R¹⁶ represent hydrogen atoms or, taken together, a double bond,
and pharmaceutically acceptable salts thereof,
in the production of drugs used to induce amenorrhea.

4. Use of compounds of general formula I 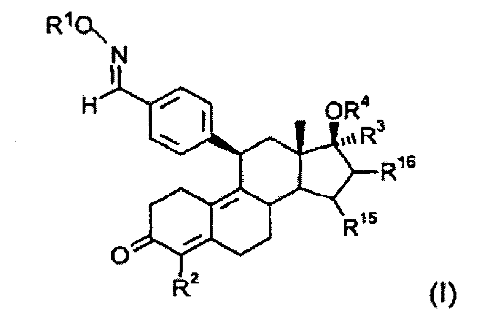 wherein the residues R¹, R², R³, R⁴, R⁵, as well as R¹⁵ and R¹⁶, have the following meanings:
R¹ represents a hydrogen atom, an alkanoyl residue with 1 to 10 carbon atoms or an optionally substituted benzoyl residue with 6-10 carbon atoms or a CONHR⁵ residue, wherein
R⁵ is a hydrogen atom, an alkyl or acyl residue with 1-10 carbon atoms, or an alkylaryl or aralkyl residue with 6-10 carbon atoms,
R² represents a hydrogen atom, a halogen atom or a CF₃ group,
R³ represents a hydrogen atom or a CH₂X group wherein
X represents a hydrogen atom, a hydroxy group, a halogen atom, an alkyl residue with 1 or 2 carbon atoms, a (CH₂)ₙCH₂Y residue wherein n = 0 or 1 and Y represents a halogen atom, and
if R² is a halogen atom, R³ additionally can be a CₙFₘHₒ group wherein n = 1, 2, 3, 4 or 5, m > 1, and m + o = 2n + 1,
R⁴ represents a hydrogen atom, an alkyl or alkanoyl residue with 1-10 carbon atoms or a benzoyl residue with 6-10 carbon atoms or a -CONHR⁵ residue wherein R⁵ has the meaning specified above,
R¹⁵ and R¹⁶ represent hydrogen atoms or, taken together, a double bond,
and pharmaceutically acceptable salts thereof,
in the production of drugs for the treatment of hormonal disorders in postmenopausal females.

5. The use according to any of claims 1 to 2 in combination with at least one low-dosed natural or synthetic estrogen or prodrugs thereof.

6. The use according to claim 5, **characterized in that** the estrogen is employed as 3-sulfamate.

7. The use according to claim 6, **characterized in that** the estrogen-3-sulfamate is 17β-hydroxyestra-1,3,5(10)-trien-3-yl sulfamate.

8. Compounds of general formula I 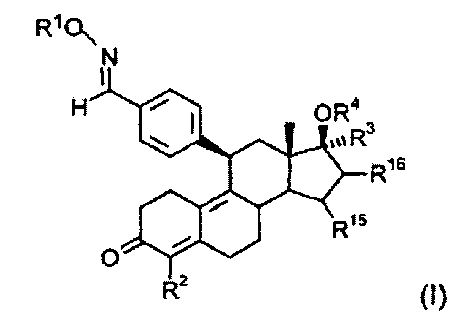 wherein the residues R¹, R², R³, R⁴, R⁵, as well as R¹⁵ and R¹⁶, have the following meanings:
R¹ represents a hydrogen atom, an alkanoyl residue with 1 to 10 carbon atoms or an optionally substituted benzoyl residue with 6-10 carbon atoms or a CONHR⁵ residue, wherein
R⁵ is a hydrogen atom, an alkyl or acyl residue with 1-10 carbon atoms, or an alkylaryl or aralkyl residue with 6-10 carbon atoms,
R² represents a halogen atom or a CF₃ group,
R³ represents a hydrogen atom or a CH₂X group wherein
X represents a hydrogen atom, a hydroxy group, a halogen atom, an alkyl residue with 1 or 2 carbon atoms, a (CH₂)ₙCH₂Y residue wherein n = 0 or 1 and Y represents a halogen atom, and
if R² is a halogen atom, R³ additionally can be a CₙFₘHₒ group wherein n = 1, 2, 3, 4 or 5, m > 1, and m + o = 2n + 1,
R⁴ represents a hydrogen atom, an alkyl or alkanoyl residue with 1-10 carbon atoms or a benzoyl residue with 6-10 carbon atoms or a -CONHR⁵ residue wherein R⁵ has the meaning specified above,
R¹⁵ and R¹⁶ represent hydrogen atoms or, taken together, a double bond,
and pharmaceutically acceptable salts thereof.

9. The compounds of general formula I according to claim 8, wherein R² is a chlorine or bromine atom.

10. The compounds of general formula I according to claim 8, wherein R³ represents a hydrogen atom or a CH₂X group, wherein
X can be a hydrogen atom, a hydroxy group, a halogen atom, an alkyl residue with 1 or 2 carbon atoms, a (CH₂)ₙCH₂Y residue wherein n = 0 or 1 and Y can be a halogen atom, and
X and/or Y can be fluorine, chlorine or bromine.

11. The compounds of general formula I according to claim 8, **characterized in that** R⁴ is a hydrogen atom or an alkyl residue with 1 to 4 carbon atoms.

12. The compounds of general formula I according to claim 8, wherein R¹ represents a hydrogen atom, R² represents a chlorine or bromine atom, and R³ represents a hydrogen atom, a methyl group, a CH₂X group wherein X represents a fluorine, chlorine or bromine atom or a hydroxy group.

13. The compounds of general formula I according to claim 8, namely:
4-[4'-bromo-17β-hydroxy-3-oxoestra-4,9-dien-11β-yl]benzaldehyde-1-(E)-oxime,
4-[4'-bromo-17β-hydroxy-17α-methyl-3-oxoestra-4,9-dien-11β-yl]-benzaldehyde-1-(E)-oxime,
4-[4'-bromo-17β-hydroxy-17α-trifluoromethyl-3-oxoestra-4,9-dien-11β-yl]benzaldehyde-1-(E)-oxime,
4-[17β-acetoxy-4'-bromo-3-oxoestra-4,9-dien-11β-yl]benzaldehyde-1-(E)-oxime,
4-[17β-acetoxy-4'-bromo-3-oxoestra-4,9-dien-11β-yl]benzaldehyde-1-(E)-O-acetyloxime,
4-[4'-bromo-17β-methoxy-3-oxoestra-4,9-dien-11β-yl]benzaldehyde-1-(E)-oxime,
4-[4'-chloro-17β-hydroxy-17α-trifluoromethyl-3-oxoestra-4,9-dien-11β-yl]benzaldehyde-1-(E)-oxime,
4-[4'-bromo-17α-fluoromethyl-17β-hydroxy-3-oxoestra-4,9-dien-11β-yl]benzaldehyde-1-(E)-oxime,
4-[4'-bromo-17α-chloromethyl-17β-hydroxy-3-oxoestra-4,9-dien-11β-yl]benzaldehyde-1-(E)-oxime,
4-[4'-bromo-17α-bromomethyl-17β-hydroxy-3-oxoestra-4,9-dien-11β-yl]benzaldehyde-1-(E)-oxime,
4-[4'-chloro-17β-methoxy-3-oxoestra-4,9-dien-11β-yl]benzaldehyde-1-(E)-oxime,
4-[4'-chloro-17α-chloromethyl-17β-hydroxy-3-oxoestra-4,9-dien-11β-yl]benzaldehyde-1-(E)-oxime,
4-[17β-methoxy-4'-trifluoromethyl-3-oxoestra-4,9-dien-11β-yl]benzaldehyde-1-(E)-oxime,
4-[4'-chloro-17β-hydroxy-17α-methyl-3-oxoestra-4,9-dien-11β-yl]benzaldehyde-1-(E)-oxime.

14. Pharmaceutical preparations including at least one compound of general formula I according to claim 8 and a pharmaceutically tolerable carrier.

15. Use of the compounds of general formula I according to any of claims 8 to 13 in the production of drugs for the treatment of endometriosis and uterus myoma.

16. Use of the compounds of general formula I according to any of claims 8 to 13 in the production of drugs for use in female fertility control.

17. Use of compounds of general formula I 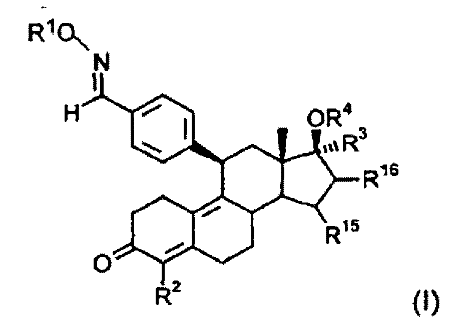 wherein the residues R¹, R², R³, R⁴, R⁵, as well as R¹⁵ and R¹⁶, have the following meanings:
R¹ represents a hydrogen atom, an alkanoyl residue with 1 to 10 carbon atoms or an optionally substituted benzoyl residue with 6-10 carbon atoms or a CONHR⁵ residue, wherein
R⁵ is a hydrogen atom, an alkyl or acyl residue with 1-10 carbon atoms, or an alkylaryl or aralkyl residue with 6-10 carbon atoms,
R² represents a hydrogen atom,
R³ represents a hydrogen atom or a CH₂X group wherein
X represents a hydrogen atom, a hydroxy group, a halogen atom, an alkyl residue with 1 or 2 carbon atoms, a (CH₂)ₙCH₂Y residue wherein n = 0 or 1 and Y represents a halogen atom, and
if R² is a halogen atom, R³ additionally can be a CₙFₘHₒ group wherein n = 1, 2, 3, 4 or 5, m > 1, and m + o = 2n + 1,
R⁴ represents a hydrogen atom, an alkyl or alkanoyl residue with 1-10 carbon atoms or a benzoyl residue with 6-10 carbon atoms or a -CONHR⁵ residue wherein R⁵ has the meaning specified above,
R¹⁵ and R¹⁶ represent hydrogen atoms or, taken together, a double bond,
and pharmaceutically acceptable salts thereof,
in the production of drugs for use in female fertility control.

18. The use according to claim 16 or 17 in combination with at least one low-dosed natural or synthetic estrogen or prodrugs thereof.

19. The use according to claim 18, **characterized in that** the estrogen is employed as 3-sulfamate.

20. The use according to claim 19, **characterized in that** the estrogen-3-sulfamate is 17β-hydroxyestra-1,3,5(10)-trien-3-yl sulfamate.

## Revendications

1. Utilisation de composés de formule générale I 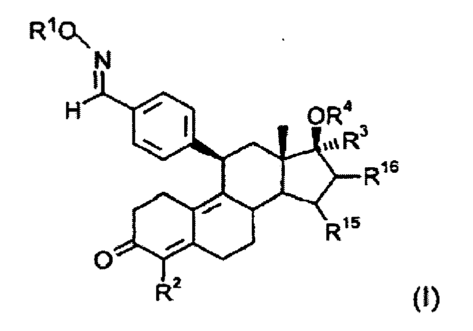 dans laquelle les radicaux R¹, R², R³, R⁴, R⁵, ainsi que R¹⁵ et R¹⁶ ont la signification suivante :
R¹ représente un atome d'hydrogène, un groupe alcanoyle comportant 1 à 10 atomes de carbone ou un groupe benzoyle éventuellement substitué comportant 6 à 10 atomes de carbone ou un groupe CONHR⁵, dans lequel
R⁵ est un atome d'hydrogène, un groupe alkyle ou acyle comportant respectivement 1 à 10 atomes de carbones ou un groupe alkylaryle ou aralkyle comportant respectivement 6 à 10 atomes de carbone,
R² représente un atome d'hydrogène, un atome d'halogène ou un groupe CF₃,
R³ représente un atome d'hydrogène ou un groupe CH₂X, dans lequel X est un atome d'hydrogène, un groupe hydroxy, un atome d'halogène, un groupe alkyle comportant 1 ou 2 atomes de carbone, un groupe (CH₂)ₙCH₂Y dans lequel n = 0 ou 1 et Y est un atome d'halogène, moyennant quoi,
si R² est un atome d'halogène, R³ peut représenter en plus un groupe CₙFₘHₒ, dans lequel n = 1, 2, 3, 4 ou 5, m > 1 et m + o = 2n + 1,
R⁴ représente un atome d'hydrogène, un groupe alkyle ou alcanoyle comportant respectivement 1 à 10 atomes de carbone ou un groupe benzoyle comportant 6 à 10 atomes de carbone ou un groupe -CONHR⁵, dans lequel R⁵ a la signification mentionnée ci-dessus,
R¹⁵ et R¹⁶ représentent des atomes d'hydrogène ou ensemble une double liaison,
et de leurs sels pharmaceutiquement compatibles,
pour préparer des médicaments destinés au traitement des saignements dysfonctionnels.

2. Utilisation de composés de formule générale I 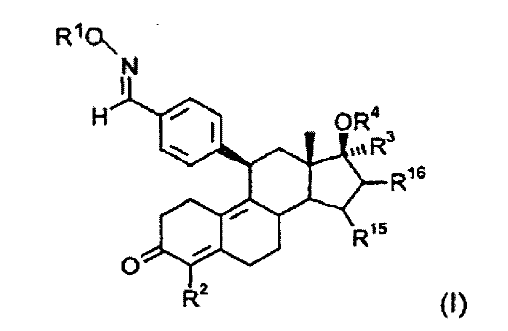 dans laquelle les radicaux R¹, R², R³, R⁴, R⁵, ainsi que R¹⁵ et R¹⁶ ont la signification suivante :
R¹ représente un atome d'hydrogène, un groupe alcanoyle comportant 1 à 10 atomes de carbone ou un groupe benzoyle éventuellement substitué comportant 6 à 10 atomes de carbone ou un groupe CONHR⁵, dans lequel
R⁵ est un atome d'hydrogène, un groupe alkyle ou acyle comportant respectivement 1 à 10 atomes de carbones ou un groupe alkylaryle ou aralkyle comportant respectivement 6 à 10 atomes de carbone,
R² représente un atome d'hydrogène, un atome d'halogène ou un groupe CF₃,
R³ représente un atome d'hydrogène ou un groupe CH₂X, dans lequel X est un atome d'hydrogène, un groupe hydroxy, un atome d'halogène, un groupe alkyle comportant 1 ou 2 atomes de carbone, un groupe (CH₂)ₙCH₂Y dans lequel n = 0 ou 1 et Y est un atome d'halogène, moyennant quoi,
si R² est un atome d'halogène, R³ peut représenter en plus un groupe CₙFₘHₒ, dans lequel n = 1, 2, 3, 4 ou 5, m > 1 et m + o = 2n + 1
R⁴ représente un atome d'hydrogène, un groupe alkyle ou alcanoyle comportant respectivement 1 à 10 atomes de carbone ou un groupe benzoyle comportant 6 à 10 atomes de carbone ou un groupe -CONHR⁵, dans lequel R⁵ a la signification mentionnée ci-dessus,
R¹⁵ et R¹⁶ représentent des atomes d'hydrogène ou ensemble une double liaison,
et de leurs sels pharmaceutiquement compatibles,
pour préparer des médicaments destinés au traitement de la dysménorrhée.

3. Utilisation de composés de formule générale I 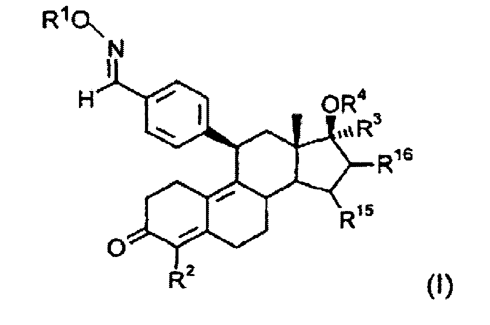 dans laquelle les radicaux R¹, R², R³, R⁴, R⁵, ainsi que R¹⁵ et R¹⁶ ont la signification suivante :
R¹ représente un atome d'hydrogène, un groupe alcanoyle comportant 1 à 10 atomes de carbone ou un groupe benzoyle éventuellement substitué comportant 6 à 10 atomes de carbone ou un groupe CONHR⁵, dans lequel
R⁵ est un atome d'hydrogène, un groupe alkyle ou acyle comportant respectivement 1 à 10 atomes de carbones ou un groupe alkylaryle ou aralkyle comportant respectivement 6 à 10 atomes de carbone,
R² représente un atome d'hydrogène, un atome d'halogène ou un groupe CF₃,
R³ représente un atome d'hydrogène ou un groupe CH₂X, dans lequel X est un atome d'hydrogène, un groupe hydroxy, un atome d'halogène, un groupe alkyle comportant 1 ou 2 atomes de carbone, un groupe (CH₂)ₙCH₂Y dans lequel n = 0 ou 1 et Y est un atome d'halogène, moyennant quoi,
si R² est un atome d'halogène, R³ peut représenter en plus un groupe CₙFₘHₒ, dans lequel n = 1, 2, 3, 4 ou 5, m > 1 et m + o = 2n + 1,
R⁴ représente un atome d'hydrogène, un groupe alkyle ou alcanoyle comportant respectivement 1 à 10 atomes de carbone ou un groupe benzoyle comportant 6 à 10 atomes de carbone ou un groupe -CONHR⁵, dans lequel R⁵ a la signification mentionnée ci-dessus,
R¹⁵ et R¹⁶ représentent des atomes d'hydrogène ou ensemble une double liaison,
et de leurs sels pharmaceutiquement compatibles, pour préparer des médicaments destinés à induire une aménorrhée.

4. Utilisation de composés de formule générale I 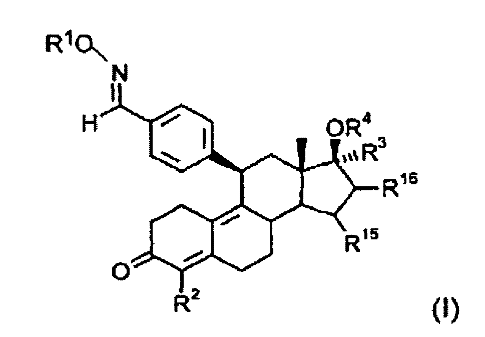 dans laquelle les radicaux R¹, R², R³, R⁴, R⁵, ainsi que R¹⁵ et R¹⁶ ont la signification suivante :
R¹ représente un atome d'hydrogène, un groupe alcanoyle comportant 1 à 10 atomes de carbone ou un groupe benzoyle éventuellement substitué comportant 6 à 10 atomes de carbone ou un groupe CONHR⁵, dans lequel
R⁵ est un atome d'hydrogène, un groupe alkyle ou acyle comportant respectivement 1 à 10 atomes de carbones ou un groupe alkylaryle ou aralkyle comportant respectivement 6 à 10 atomes de carbone,
R² représente un atome d'hydrogène, un atome d'halogène ou un groupe CF₃,
R³ représente un atome d'hydrogène ou un groupe CH₂X, dans lequel X est un atome d'hydrogène, un groupe hydroxy, un atome d'halogène, un groupe alkyle comportant 1 ou 2 atomes de carbone, un groupe (CH₂)ₙCH₂Y dans lequel n = 0 ou 1 et Y est un atome d'halogène, moyennant quoi,
si R² est un atome d'halogène, R³ peut représenter en plus un groupe CₙFₘHₒ, dans lequel n = 1, 2, 3, 4 ou 5, m > 1 et m + o = 2n + 1,
R⁴ représente un atome d'hydrogène, un groupe alkyle ou alcanoyle comportant respectivement 1 à 10 atomes de carbone ou un groupe benzoyle comportant 6 à 10 atomes de carbone ou un groupe -CONHR⁵, dans lequel R⁵ a la signification mentionnée ci-dessus,
R¹⁵ et R¹⁶ représentent des atomes d'hydrogène ou ensemble une double liaison,
et de leurs sels pharmaceutiquement compatibles,
pour préparer des médicaments destinés au traitement des troubles hormonaux chez les femmes ménopausées.

5. Utilisation selon une des revendications 1 à 2 en association avec au moins un oestrogène naturel ou synthétique microdosé ou leurs précurseurs pharmacologiques.

6. Utilisation selon la revendication 5, **caractérisée en ce qu'**on introduit l'oestrogène sous forme de 3-sulfamate.

7. Utilisation selon la revendication 6, **caractérisée en ce que** l'oestrogène-3-sulfamate est du 17β-hydroxy-estra-1,3,5(10)-trién-3yl-sulfamate.

8. Composés de formule générale I 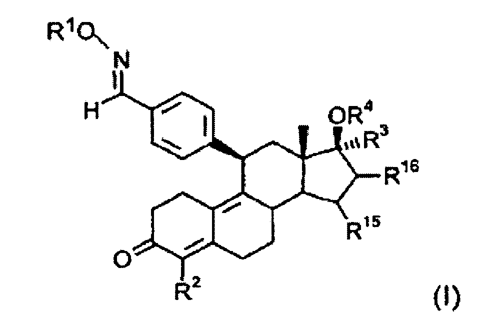 dans laquelle les radicaux R¹, R², R³, R⁴, R⁵, ainsi que R¹⁵ et R¹⁶ ont la signification suivante :
R¹ représente un atome d'hydrogène, un groupe alcanoyle comportant 1 à 10 atomes de carbone ou un groupe benzoyle éventuellement substitué comportant 6 à 10 atomes de carbone ou un groupe CONHR⁵, dans lequel
R⁵ est un atome d'hydrogène, un groupe alkyle ou acyle comportant respectivement 1 à 10 atomes de carbones ou un groupe alkylaryle ou aralkyle comportant respectivement 6 à 10 atomes de carbone,
R² représente un atome d'halogène ou un groupe CF₃,
R³ représente un atome d'hydrogène ou un groupe CH₂X, dans lequel X est un atome d'hydrogène, un groupe hydroxy, un atome d'halogène, un groupe alkyle comportant 1 ou 2 atomes de carbone, un groupe (CH₂)ₙCH₂Y dans lequel n = 0 ou 1 et Y est un atome d'halogène, moyennant quoi,
si R² est un atome d'halogène, R³ peut représenter en plus un groupe CₙFₘHₒ, dans lequel n = 1, 2, 3, 4 ou 5, m > 1 et m + o = 2n + 1,
R⁴ représente un atome d'hydrogène, un groupe alkyle ou alcanoyle comportant respectivement 1 à 10 atomes de carbone ou un groupe benzoyle comportant 6 à 10 atomes de carbone ou un groupe -CONHR⁵, dans lequel R⁵ a la signification mentionnée ci-dessus,
R¹⁵ et R¹⁶ représentent des atomes d'hydrogène ou ensemble une double liaison,
et leurs sels pharmaceutiquement compatibles,

9. Composés de formule générale I selon la revendication 8, dans laquelle R² est un atome de chlore ou de brome.

10. Composés de formule générale I selon la revendication 8, dans laquelle R³ est un atome d'hydrogène ou un groupe CH₂X, dans lequel
X peut être un atome d'hydrogène, un groupe hydroxy, un atome d'halogène, un groupe alkyle comportant 1 à 2 atomes de carbone, un groupe (CH₂)ₙCH₂Y dans lequel n = 0 ou 1 et Y est un atome d'halogène, et X et/ou Y peuvent être du fluor, du chlore ou du brome.

11. Composés de formule générale I selon la revendication 8, **caractérisés en ce que** R⁴ est un atome d'hydrogène ou un groupe alkyle comportant 1 à 4 atomes de carbone.

12. Composés de formule générale I selon la revendication 8, dans laquelle R¹ représente un atome d'hydrogène, R² représente un atome de chlore ou de brome et R³ peut être un atome d'hydrogène, un groupe méthyle, un groupe CH₂X, dans lequel X représente un atome de fluor, de chlore ou de brome ou un groupe hydroxy.

13. Composés de formule générale I selon la revendication 8, à savoir :
4-[4'-bromo-17β-hydroxy-3-oxoestra-4,9-dién-11β-yl]benzaldéhyde-1-(E)-oxime,
4-[4'-bromo-17β-hydroxy-17α-méthyl-3-oxoestra-4,9-dién-11β-yl]benzaldéhyde-1-(E)-oxime,
4-[4'-bromo-17β-hydroxy-17α-trifluorométhyl-3-oxoestra-4,9-dién-11β-yl]benzaldéhyde-1-(E)-oxime,
4-[17β-acétoxy-4'-bromo-3-oxoestra-4,9-dién-11β-yl]benzaldéhyde-1-(E)-oxime,
4-[17β-acétoxy-4'-bromo-3-oxoestra-4,9-dién-11β-yl]benzaldéhyde-1-(E)-O-acétyloxime,
4-[4'-bromo-17β-méthoxy-3-oxoestra-4,9-dién-11β-yl]benzaldéhyde-1-(E)-oxime,
4-[4'-chloro-17β-hydroxy-17α-trifluorométhyl-3-oxoestra-4,9-dién-11β-yl]benzaldéhyde-1-(E)-oxime,
4-[4'-bromo-17α-fluorométhyl-17β-hydroxy-3-oxoestra-4,9-dién-11β-yl]benzaldéhyde-1-(E)-oxime,
4-[4'-bromo-17α-chlorométhyl-17β-hydroxy-3-oxoestra-4,9-dién-11β-yl]benzaldéhyde-1-(E)-oxime,
4-[4'-bromo-17α-bromométhyl-17β-hydroxy-3-oxoestra-4,9-dién-11β-yl]benzaldéhyde-1-(E)-oxime,
4-[4'-chloro-17β'-méthoxy-3-oxoestra-4,9-dién-11β-yl]benzaldéhydé-1-(E)-oxime,
4-[4'-chloro-17α-chlorométhyl-17β-hydroxy-3-oxoestra-4,9-dién-11β-yl]benzaldéhyde-1-(E)-oxime,
4-[17β-méthoxy-4'-trifluorométhyl-3-oxoestra-4,9-dién-11β-yl]benzaldéhyde-1-(E)-oxime,
4-[4'-chloro-17β-hydroxy-17α-méthyl-3-oxoestra-4,9-dién-11β-yl]benzaldéhyde-1-(E)-oxime.

14. Préparations pharmaceutiques contenant au moins un composé de formule générale I selon la revendication 8 ainsi qu'un vecteur pharmaceutiquement compatible.

15. Utilisation des composés de formule générale I selon l'une des revendications 8 à 13 pour préparer des médicaments destinés au traitement de l'endométriose ainsi que de la myomatose de l'utérus.

16. Utilisation des composés de formule générale I selon l'une des revendications 8 à 13 pour préparer des médicaments destinés au contrôle de la fertilité féminine.

17. Utilisation de composés de formule générale I 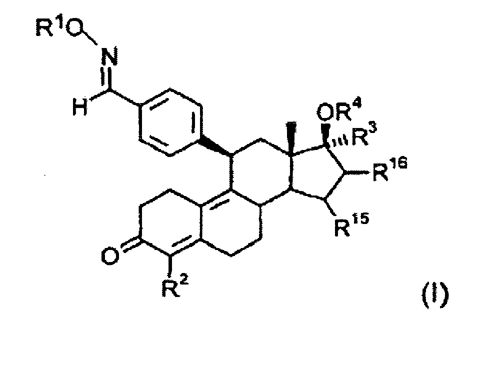 dans laquelle les radicaux R¹, R², R³, R⁴, R⁵, ainsi que R¹⁵ et R¹⁶ ont la signification suivante :
R¹ représente un atome d'hydrogène, un groupe alcanoyle comportant 1 à 10 atomes de carbone ou un groupe benzoyle éventuellement substitué comportant 6 à 10 atomes de carbone ou un groupe CONHR⁵, dans lequel
R⁵ est un atome d'hydrogène, un groupe alkyle ou acyle comportant respectivement 1 à 10 atomes de carbones ou un groupe alkylaryle ou aralkyle comportant respectivement 6 à 10 atomes de carbone,
R² représente un atome d'hydrogène,,
R³ représente un atome d'hydrogène ou un groupe CH₂X, dans lequel X est un atome d'hydrogène, un groupe hydroxy, un atome d'halogène, un groupe alkyle comportant 1 ou 2 atomes de carbone, un groupe (CH₂)ₙCH₂Y dans lequel n = 0 ou 1 et Y est un atome d'halogène, moyennant quoi,
si R² est un atome d'halogène, R³ peut représenter en plus un groupe CₙFₘHₒ, dans lequel n = 1, 2, 3, 4 ou 5, m > 1 et m + o = 2n + 1,
R⁴ représente un atome d'hydrogène, un groupe alkyle ou alcanoyle comportant respectivement 1 à 10 atomes de carbone ou un groupe benzoyle comportant 6 à 10 atomes de carbone ou un groupe -CONHR⁵, dans lequel R⁵ a la signification mentionnée ci-dessus,
R¹⁵ et R¹⁶ représentent des atomes d'hydrogène ou ensemble une double liaison,
et de leurs sels pharmaceutiquement compatibles
pour préparer des médicaments destinés au contrôle de la fertilité féminine.

18. Utilisation selon une des revendication 16 ou 17 en association avec au moins un oestrogène naturel ou synthétique microdosé ou leurs précurseurs pharmacologiques.

19. Utilisation selon la revendication 18, **caractérisée en ce qu'**on introduit l'oestrogène sous forme de 3-sulfamate.

20. Utilisation selon la revendication 19, **caractérisée en ce que** l'oestrogène-3-sulfamate est du 17β-hydroxy-estra-1,3,5(10)-trién-3yl-sulfamate.
